# EUROPEAN PATENT SPECIFICATION

(11) **EP 1 896 849 B1**
(45) Date of publication and mention of the grant of the patent: **24.11.2010**
(21) Application number: 06752069.2
(22) Date of filing: 01.05.2006
(51) Int. Cl.: G01N 33/53, A61K 39/395, C07K 16/00

(54) **ANTIBODIES AGAINST HISTONE MODIFICATIONS FOR CLINICAL DIAGNOSIS AND PROGNOSIS OF CANCER**
ANTIKÖRPER GEGEN HISTONMODIFIKATIONEN FÜR DIE KLINISCHE KREBSDIAGNOSE UND -PROGNOSE
ANTICORPS DIRIGÉS CONTRE DES MODIFICATIONS DES HISTONES POUR LE DIAGNOSTIC ET LE PRONOSTIC CLINIQUES D'UN CANCER

(30) Priority: 29.04.2005 US 676021 P; 01.03.2006 US 778235 P
(43) Date of publication of application: 12.03.2008
(60) Divisional application: 10009991.0
(73) Proprietor: The Regents of The University of California, Oakland, CA 94607-5200 (US)
(72) Inventor: KURDISTANI, Siavash K., Los Angeles, California 90025 (US); GRUNSTEIN, Michael, Los Angeles, California 90049 (US); SELIGSON, David B., Los Angeles, California 90066 (US)
(74) Representative: UEXKÜLL & STOLBERG
(86) International application number: PCT/US2006/016765
(87) International publication number: WO 2006/119264

(56) References cited:
- EP-A- 1 403 639
- WO-A-03/014142
- WO-A-03/070894
- WO-A-2005/019826
- US-A1- 2003 170 689
- US-A1- 2005 069 931
- KANG Z ET AL: "COREGULATOR RECRUITMENT AND HISTONE MODIFICATIONS IN TRANSCRIPTIONAL REGULATION BY THE ANDROGEN RECEPTOR" MOLECULAR ENDOCRINOLOGY, BALTIMORE, MD, US, vol. 18, no. 11, 1 November 2004 (2004-11-01), pages 2633-2648, XP009045419 ISSN: 0888-8809
- KONDO YUTAKA ET AL: "Critical role of histone methylation in tumor suppressor gene silencing in colorectal cancer" MOLECULAR AND CELLULAR BIOLOGY, AMERICAN SOCIETY FOR MICROBIOLOGY, WASHINGTON, US, vol. 23, no. 1, 1 January 2003 (2003-01-01), pages 206-215, XP002308761 ISSN: 0270-7306
- KONDO Y ET AL: "Chromatin immunoprecipitation microarrays for identification of genes silenced by histone H3 lysine 9 methylation" PROCEEDINGS OF THE NATIONAL ACADEMY OF SCIENCES OF USA, NATIONAL ACADEMY OF SCIENCE, WASHINGTON, DC.; US, vol. 101, no. 19, 11 May 2004 (2004-05-11), pages 7398-7403, XP002996405 ISSN: 0027-8424
- NGUYEN CARVELL T ET AL: "Histone H3-lysine 9 methylation is associated with aberrant gene silencing in cancer cells and is rapidly reversed by 5-aza-2'-deoxycytidine" CANCER RESEARCH, AMERICAN ASSOCIATION FOR CANCER RESEARCH, BALTIMORE, MD.; US, vol. 62, no. 22, 15 November 2002 (2002-11-15), pages 6456-6461, XP002458349
- KONDO YUTAKA ET AL: "Contribution of histone H3 lysine 9 and 27 methyltransferases to gene silencing and neoplastic phenotype in cancer." PROCEEDINGS OF THE AMERICAN ASSOCIATION FOR CANCER RESEARCH ANNUAL MEETING, vol. 46, 1 April 2005 (2005-04-01), page 642, XP002506082 & 96TH ANNUAL MEETING OF THE AMERICAN-ASSOCIATION-FOR-CANCER-RESEARCH; ANAHEIM, CA, USA; APRIL 16 -20, 2005 ISSN: 0197-016X Retrieved from the Internet: URL:HTTP://WWW.AACRMEETINGABSTRACTS.ORG/CG I/CONTENT/ABSTRACT/2005/1/642-B> [retrieved on 2008-11-28]
- COOMBES M M ET AL: "Resetting the histone code at CDKN2A in HNSCC by inhibition of DNA methylation" ONCOGENE 20031204 GB, vol. 22, no. 55, 4 December 2003 (2003-12-04), pages 8902-8911, XP002506083 ISSN: 0950-9232
- STIRZAKER C. ET AL.: 'Transcriptional Gene Silencing Promotes DNA Hypermethylation through a Sequential Change in Chromatin Modifications' CANCER RES vol. 64, 01 June 2004, pages 3871 - 3877, XP003005371

## Description

### BACKGROUND OF THE INVENTION

Cancer of the prostate exhibits a heterogeneous clinical behavior from indolent to highly aggressive and is the second leading cause of cancer deaths in men in the US (Jemal, et al., CA Cancer J. Clin. 53:5-26 (2003)). Current biomarkers including pre-operative PSA and biopsy Gleason score (Gleason, Cancer Chemother. Rep. 50:125-8 (1966)) (a measure of tumor differentiation, scored from 2-10 with increasing degree of de-differentiation) have not proven to be accurate predictors of clinical outcome (Bunting, Clin. Chim. Acta 315:71-97 (2002)). The lack of prognostic markers is even more pressing in younger men with asymptomatic low-grade (Gleason score <7) tumors who are being increasingly identified by prevalent screening of PSA levels, but it is unclear how aggressively they should be treated (Han, et al., J. Urol. 166:41679 (2001); Farkas, et al., Urology 52:444-8; discussion 448-9 (1998)). Therefore, improved prognostic markers are clearly needed for this and other cancers, such as breast, lung, kidney, and colon cancer.

Aberrations in histone modifications occur in human disease, including cancer. Aberrations in post-translational modifications of histones have been shown to occur in cancer cells but only at individual promoters (Jacobson, et al., Curr. Opin. Genet. Dev. 9:175-84 (1999)) and have not been related to clinical outcome. These aberrations may occur locally at promoters by inappropriate targeting of histone modifying enzymes, leading to improper expression or repression of individual genes that play important roles in tumorigenesis. However, despite a large number of genes examined, little similarity in local, gene-targeted histone modification changes in different cancers is reported. Aberrant modification of histones associated with DNA repetitive sequences has also been reported. These aberrations include lower levels of histone H4 K16Ac and K20diMe in hematological malignancies and colorectal adenocarcinomas. None of these changes, either at individual genes or at repetitive DNA elements, however, has been related to clinical outcome.

Histone modifications, such as acetylation and methylation of lysines (K) and arginines (R), which also occur over large regions of chromatin including non-promoter sequences, are referred to as global histone modifications (Vogelauer, et al., Nature 408:495-8 (2000)). As noted above, enzymes that modify histones exhibit altered activity in cancer. For instance, missense mutations of p300 histone acetyltransferases and loss of heterozygosity at the p300 locus are associated with colorectal and breast cancers, and glioblastomas (Giles, et al., Trends. Genet. 14:178-83 (1998); Gayther, et al., Nat. Genet. 24:300-3 (2000); Muraoka, et al., Oncogene 12:1565-9 (1996)). The consequence of the altered activity of histone-modifying enzymes has so far been linked to inappropriate expression of few genes that may play a role in tumor biology. For instance, p300 is involved in androgen receptor transactivation, potentially playing an important role in progression of prostate cancer (Debes, et al. Cancer Res. 63:7638-40 (2003))". Kang et al., Mol. Endocrinology 18(11):2633-2648 (2004) reported that covalent histone modifications such as acetylation (H3-K9/K14) and phosphorylation (H3-S10) accompany transcriptional activation of prostate-specific antigen and kallikrein (KLK2) genes by androgen receptor (AR). WO 2005/019826 discloses a method of assessing a disease condition in an individual by detecting histone modifications (methylation, acetylation and phosphorylation). EP 1 403 639 relates to a method for determining whether a treatment of a disorder, in particular cancer, with an HDAC inhibitor is to be started/continued or not comprising determining the level of histone acetylation in a tissue sample.

However, in addition to being targeted to promoters, these enzymes also affect most nucleosomes throughout the genome independently of apparent sequence-specific DNA binding proteins (Vogelauer, et al., Nature 408:495-8 (2000); Reid, et al., Mol. Cell 6, 1297-307 (2000); Krebs, et al., Cell 102, 587-98 (2000)).. Furthermore, the histone modifying enzymes possess a high degree of substrate specificity which differentiate between the histone sub-types as well as individual side-chains within each histone (Peterson, et al., Curr. Biol. 14, R546-51 (2004); Suka, et al., Mol. Cell 8:473-9 (2001)). Thus, individual residues will be modified globally to varying extent, reflecting the selective but widespread activity of the histone-modifying enzymes.

The present invention relates to our surprising discovery that certain global histone modifications are useful markers in the diagnosis and prognosis of cancers, such as prostate, breast, lung, kidney, and colon cancer.

### BRIEF SUMMARY OF THE INVENTION

In one aspect, the present invention provides a method of diagnosing a cancer, the method comprising (a) contacting a test tissue sample from an individual at risk of having a cancer or suspected of having a cancer or being evaluated for the possible presence of a cancer with an antibody that specifically binds to a modified histone protein; and (b) determining the global H3K4 dimethylation and H3K18 acetylation histone modification pattern in the test tissue sample in comparison to a control tissue sample; thereby diagnosing said cancer by identification of an altered global histone modification pattern.

In another aspect, the invention provides a method of prognosis for a cancer, the method comprising (a) contacting a test tissue sample from an individual at risk for or known to have a cancer with an antibody that specifically binds to a modified histone protein; and (b) determining the global H3K4 dimethylation and H3K18 acetylation histone modification pattern in the test tissue sample in comparison to a control tissue sample; thereby providing a prognosis for said cancer by identification of an altered global histone modification pattern.

In still another aspect, the invention provides a method of identifying a global histone modification pattern for the diagnosis or prognosis of cancer, said method comprising (a) contacting a test tissue sample from an individual having the cancer with an antibody that specifically binds to a modified histone protein; (b) contacting a control tissue sample from an individual not known to have the cancer with an antibody that specifically binds to the modified histone protein; and (c) determining the global H3K4 dimethylation and H3K18 acetylation histone modification pattern of the modified histone protein in the test tissue sample in comparison to the control tissue sample; thereby identifying an altered global histone modification pattern useful in the diagnosis or prognosis of cancer.

In a further aspect, the invention provides a method of predicting the likelihood of a recurrence of cancer, said method comprising (a) contacting a test tissue sample from the patient with an antibody that specifically binds to a modified histone protein; and (b) determining the global H3K4 dimethylation and H3K18 acetylation histone modification pattern in the test tissue sample in comparison to a control tissue sample or nonmalignant tissue sample; thereby predicting the likelihood of a recurrence of cancer by identification of an altered global histone modification pattern.

In preferred embodiments, the tissue sample is a tumor biopsy sample. In some embodiments, the cancer is bladder, kidney, ovarian, colon or breast cancer; it is preferred that the cancer is prostate cancer or adenocarcinoma. In some embodiments the cancer is metastatic. It is preferred that the individual has less advanced disease (i.e. low grade or stage), a category in which diagnostic markers are actually needed. In some embodiments a monoclonal antibody is used.

In another aspect, the invention provides a kit comprising at least two different antibodies which each selectively bind to a different histone modification which has a global histone modification pattern which is altered in cancer and instructions on how to relate the global histone modification to the diagnosis or prognosis of the cancer, wherein the antibodies bind the H3 K4 dimethylation and H3K18 acetylation histon protein modifications.

### BRIEF DESCRIPTION OF THE DRAWINGS

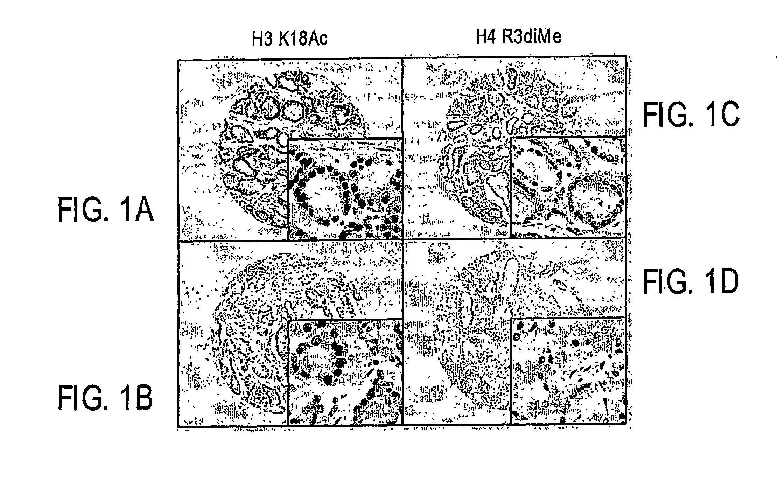

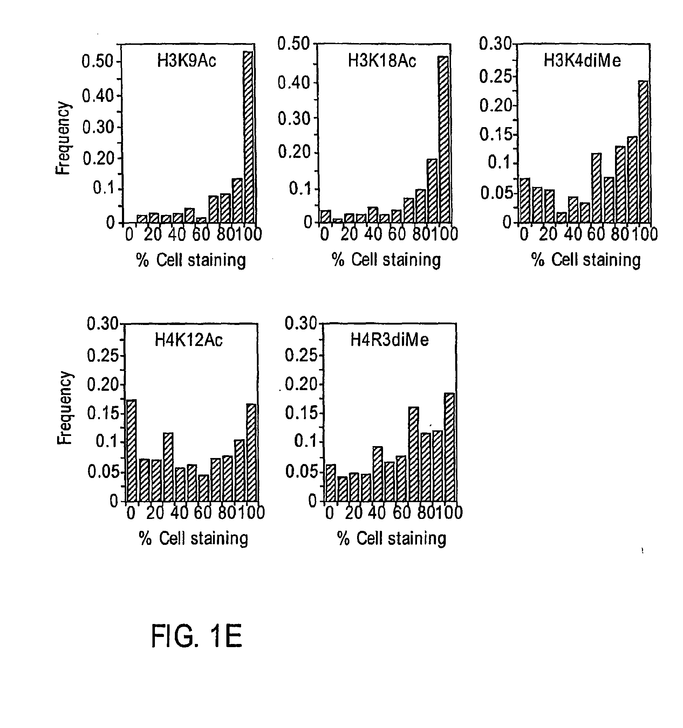

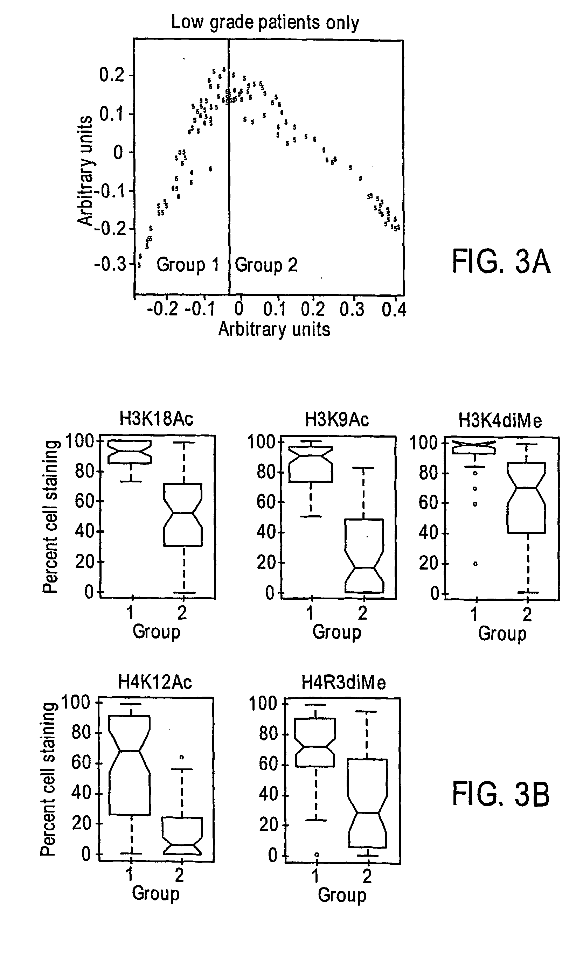

**Figure 1****:** Global levels of individual histone modifications is determined by immunohistochemistry. Characteristic nuclear staining of malignant prostate glandular cells by immunohistochemistry using anti-H3 K18 acetylation **(a, b)** or anti-H4 R3 dimethylation **(c, d**) antibodies. Representatives of group 1 patients with **(a)** 95%, **(c)** 70% and of group 2 patients with **(b)** 50% and **(d)** 25% positive staining are shown (see Fig. 3). Original objectives: X10, insets: X40. **e,** Distribution of staining for the 5 different antibodies across all 183 tissue samples. Y-axis is the fraction of samples that shows positive staining for the indicated percent of cells (X-axis).

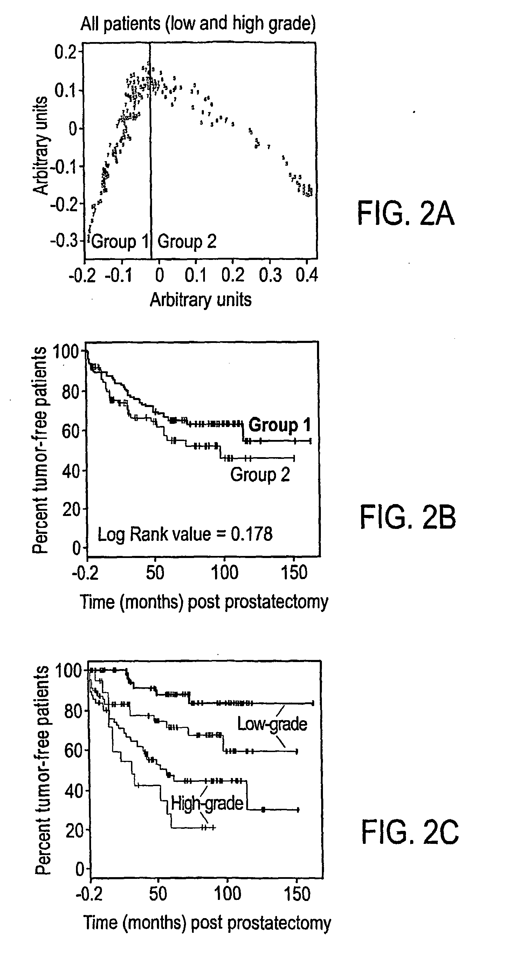

**Figure 2****:** Grouping of patients with similar histone modification patterns. **a**, An MDS plot is used to visualize the degree of dissimilarity (distance along the axes with arbitrary units) between all patients as generated by the RF algorithm. Patients are indicated by their Gleason score. Two groups are identified by inspection (blue line). **b**, Kaplan-Meier recurrence-free plots of the two groups (black-group 1; red-group 2) identified among all patients based on the histone modification patterns. **c**, Kaplan-Meier recurrence-free survival plots based on modification pattern grouping (as in **a)** and grade stratification (Low-grade patients-blue and red lines; High-grade patients-green and black lines).

**Figure 3****:** Grouping of low-grade patients with similar histone modification patterns. **a**, An MDS plot visualizes the degree of dissimilarity between patients with low grade tumors as generated by the RF algorithm. Patients are indicated by their Gleason score. Two groups are identified by inspection (blue line). **b**, Distribution of staining for the five histone modifications in group 1 (black) and group 2 (red) patients is shown as boxplots. The line in the center of each box represents the median value of the distribution and the upper and lower ends of the box are the upper (25th) and lower (75th) quartiles, respectively. The "whiskers" extend to smallest and largest observations.

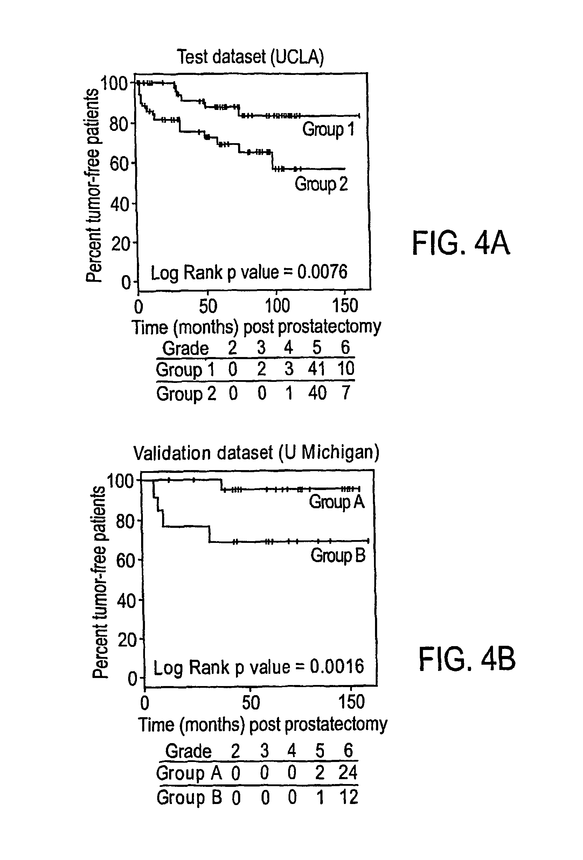

**Figure 4****:** Histone modification patterns predict tumor recurrence. **a**, Kaplan-Meier recurrence-free plots of the two groups (black - group 1; red - group 2) identified among the low-grade patients (*n*=104, UCLA) based on the histone modification patterns. Shown below is the distribution of the patients in each group according to their grade (Gleason score). **b**, Kaplan-Meier recurrence-free plots of the two groups (black-group A; red-group B) identified among the low-grade patients of the validation dataset (*n*=39, University of Michigan). The two groups are identified based on the "simple rule" involving only H3 K18Ac and H3 K4diMe modifications. Distribution of the patients in each group according to their grade is shown.

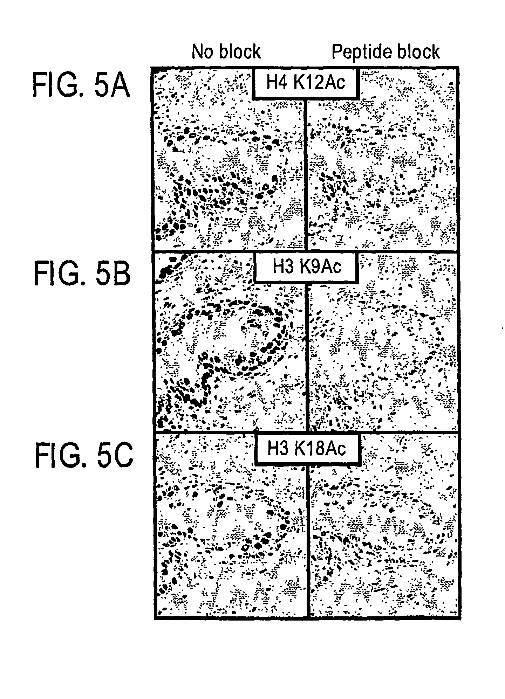

**Figure 5****:** Histone modification antibodies are specific in immunohistochemistry. Antibodies were diluted in PBS with 5% goat serum, incubated for 4 hours with no or excess antigen (singly acetylated peptide) pre-adsorbed to a nitrocellulose membrane, and used for staining. Anti-H4 K12Ac -H3K9Ac and -H3 K18Ac antibodies (a-c, respectively) are cleared by their respective antigens (right panels). Anti-H3 K4diMe (Upstate) and H4-R3dimethyl (abcam) are indicated to be specific by the manufacturer.

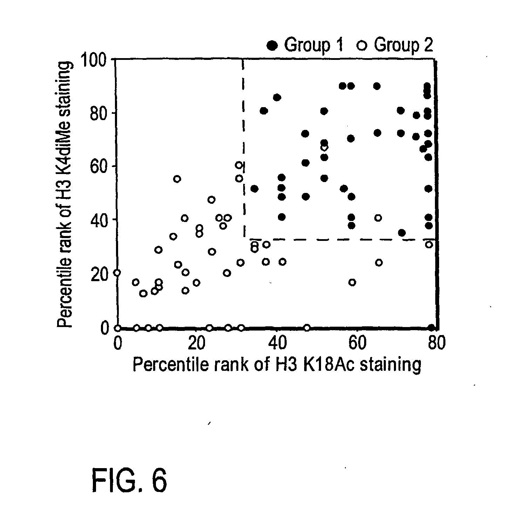

**Figure 6****:** A "simple rule" involving H3 K4diMe and H3 K18Ac estimates the grouping of patients based on Random Forests clustering of all five histone modifications. Staining above 60 percentile for H3K4diMe or above 35 percentile for H3 K18Ac and above 35 percentile staining for H3 K4diMe identifies Group 1 patients from Group 2 patients (red circles) with two misclassifications. Note that the percentile reflects the relative standing of values in a dataset. Recall from Fig 4a that group 1 patients have a lower risk of tumor recurrence compared to group 2 patients. When the rule is used to predict recurrence risk, the log rank p value becomes p = 0.028 *hazard ratio (2.8, CI = 1.12 to 7.02).. Compare to Figure 4a.

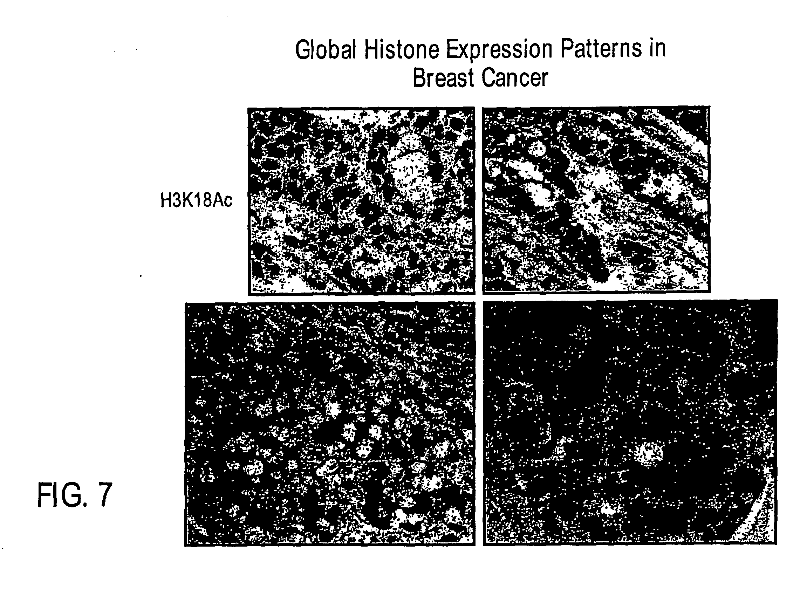

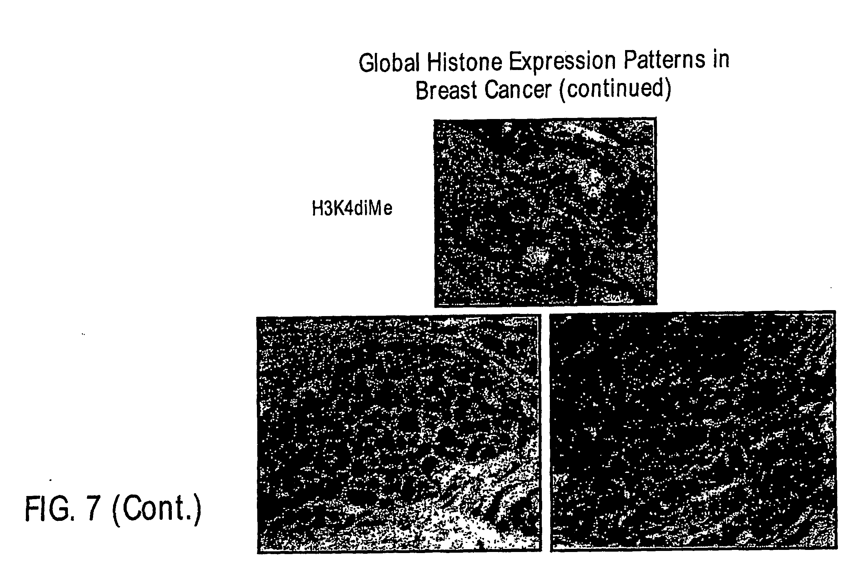

**Figure 7****:** Global histone expression patterns in breast cancer

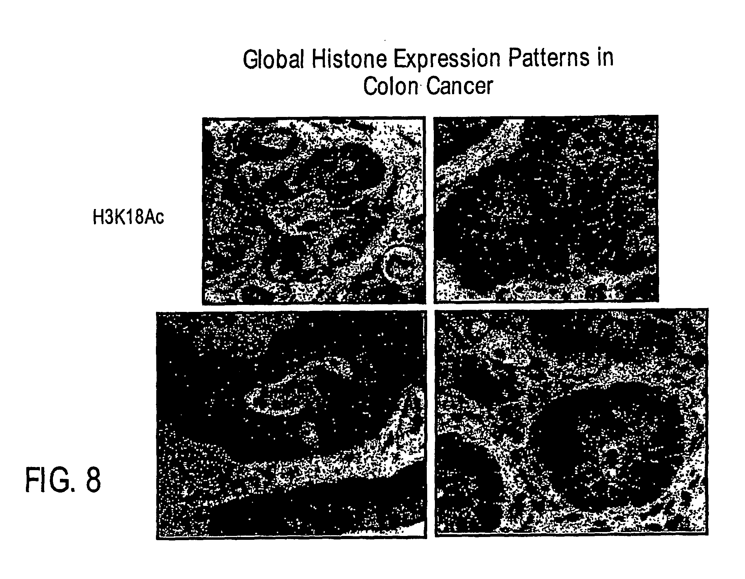

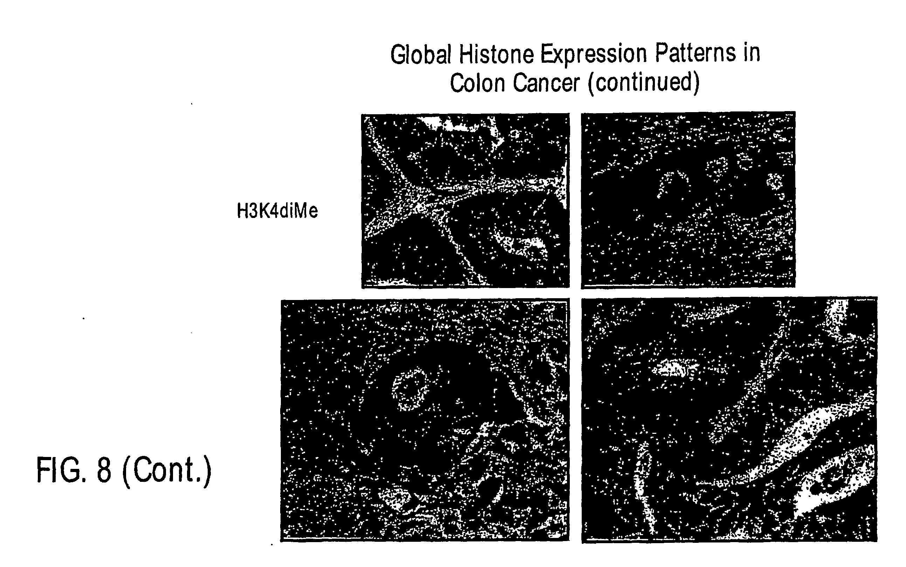

**Figure 8****:** Global histone expression patterns in colon cancer.

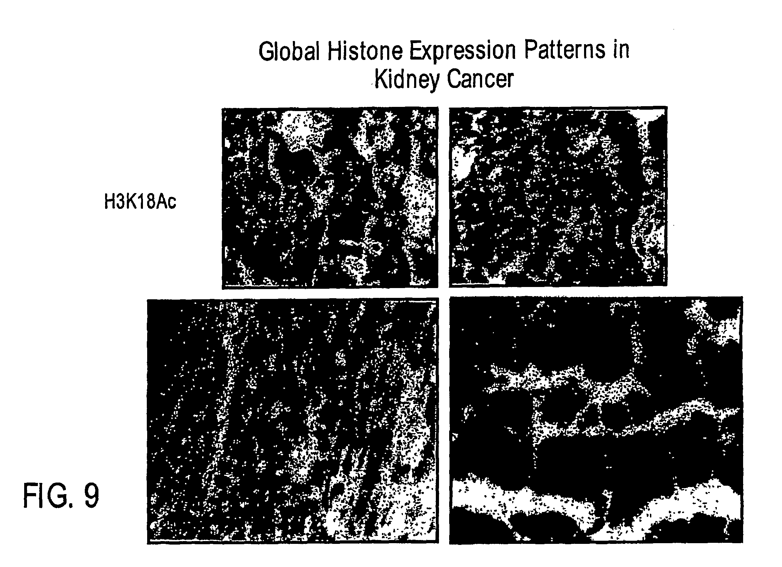

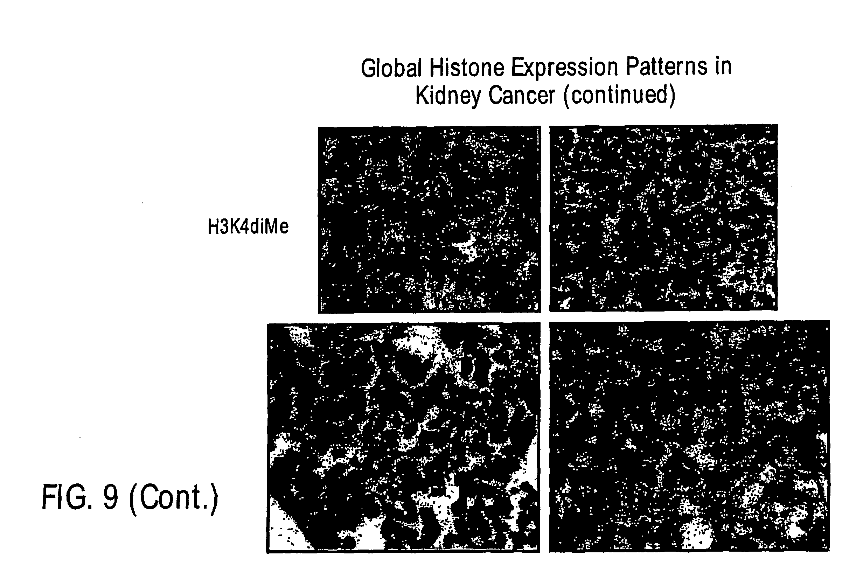

**Figure 9****:** Global histone expression patterns in kidney cancer.

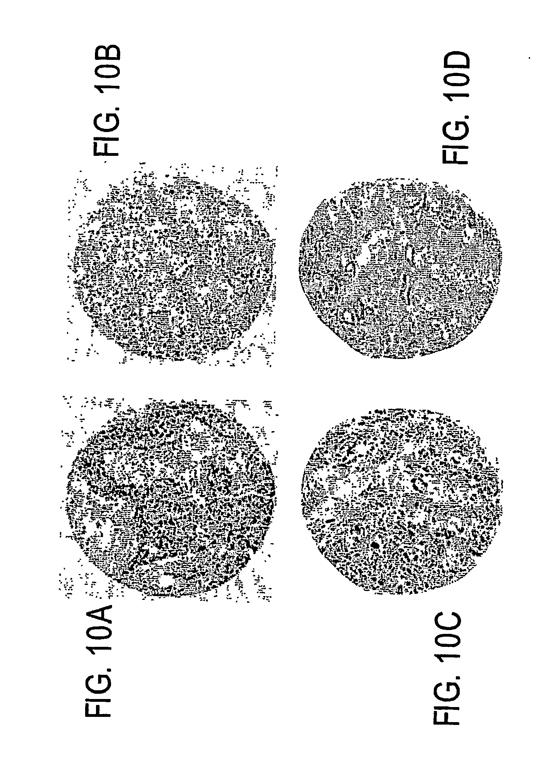

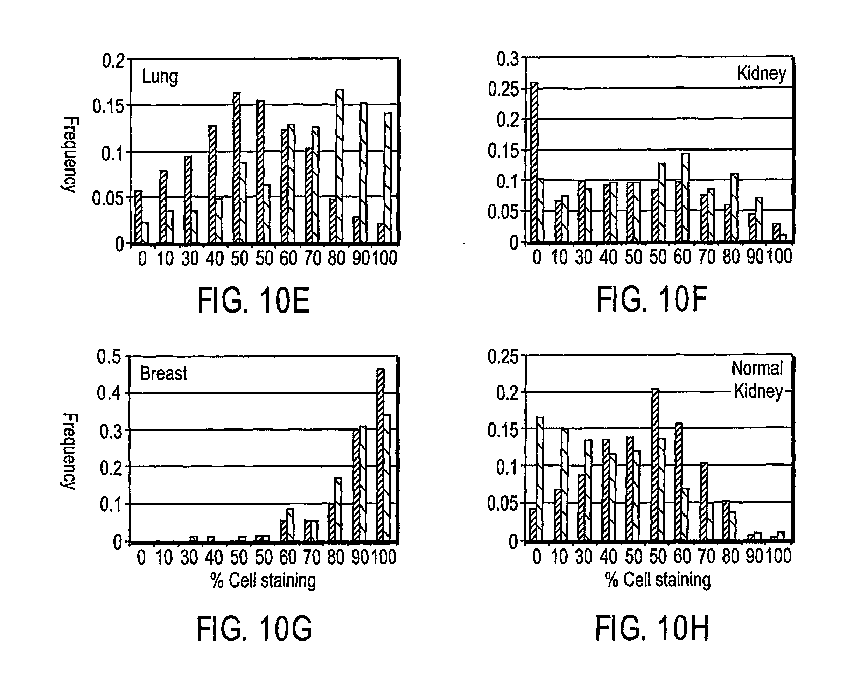

**Figure 10****:** Immunohistochemical detection and distribution of histone modifications in primary tissues. a-d Immunohistochemical staining of cancer tissues from (a) lung, (b) kidney, (c) breast and (d) matched normal tissue from kidney with anti-H3 K18 acetylation antibody. Percentage of cancer cells with brown nuclei determines the global levels of each histone modification for a given individual. Original magnification, X10; insets, X40. e-h Distribution of patients with indicated percent cell staining for H3 K4diMe (black bars) and K18Ac (grey bars) in cancer tissues from (e) lung, (f) kidney, (g) breast and (h) matched normal tissues from kidney.

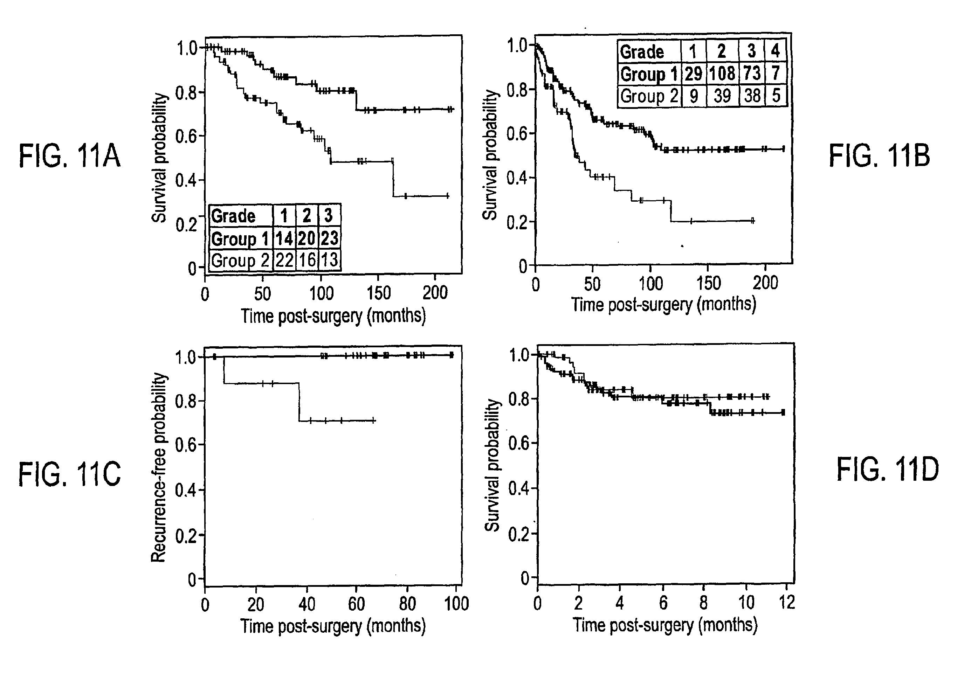

**Figure 11****.** A histone modification pattern in cancer but not normal tissues predicts clinical outcome in different carcinomas. a-c For each cancer type, patients are first assigned to two groups based on the levels of H3 K4diMe and K18Ac and their clinical outcomes are compared. Kaplan-Meir plots are used to visualize survival probabilities of the two groups (Group 1, black line; Group2, red line) in (a) lung (p=0.0034), (b) kidney (p=0.0017) and recurrence-free time in (c) breast cancer patients (p=0.006). Tabulated in the inset boxes is the distribution of the patients in each group according to their grade. (d) Grouping of kidney cancer patients based on levels of histone modifications in their matched normal tissues reveals no significant difference in survival probabilities of the two groups (p=0.61).

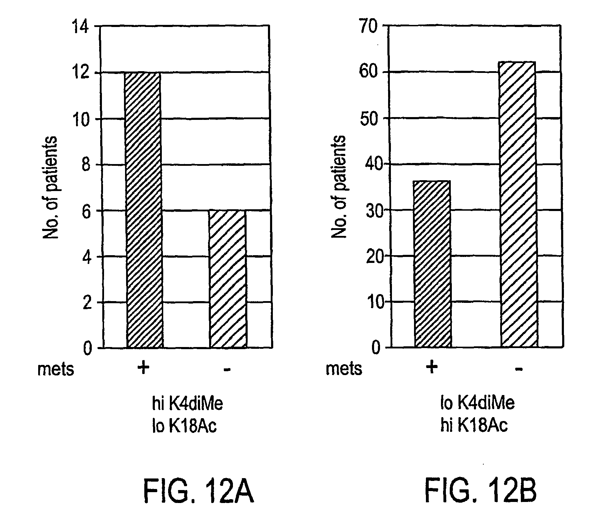

**Figure 12****.** High levels of H3 K4diMe and low levels of H3 K18Ac are associated with increased risk of metastasis in kidney cancer. Subsets of patients with (a) >60 and <35 percentile staining for H3 K4diMe and K18Ac, respectively (Group K4, n=18) and (b) <60 and >35 percentile staining for H3 K4diMe and K18Ac, respectively (Group K18, n=98) are analyzed for number of patients who had metastatic disease at the time of diagnosis. Patients in Group K4 have ∼2-fold increase in risk of metastasis when compared to Group K18 (67% vs. 37%, p=0.021).

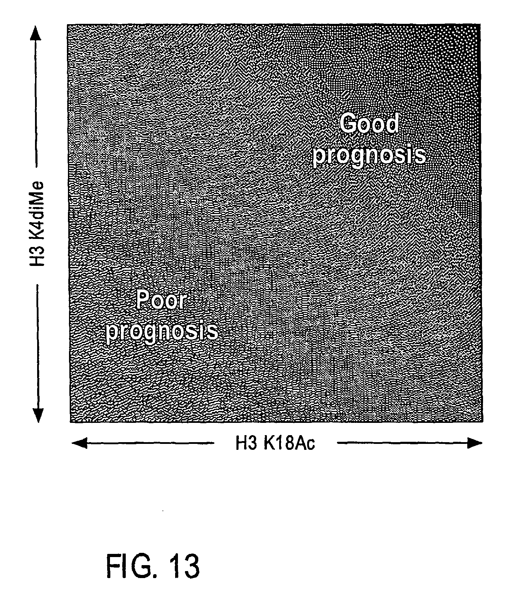

**Figure 13****.** A model for prognostication of cancer patients based on epigenetic heterogeneity. Increased prevalence of cancer cells with high levels of H3 K4diMe and K18Ac are associated with better clinical outcome (darkerareas) compared to lower levels of both modifications (lighter areas) in four different carcinomas of lung, kidney, breast and previously reported prostate cancer.

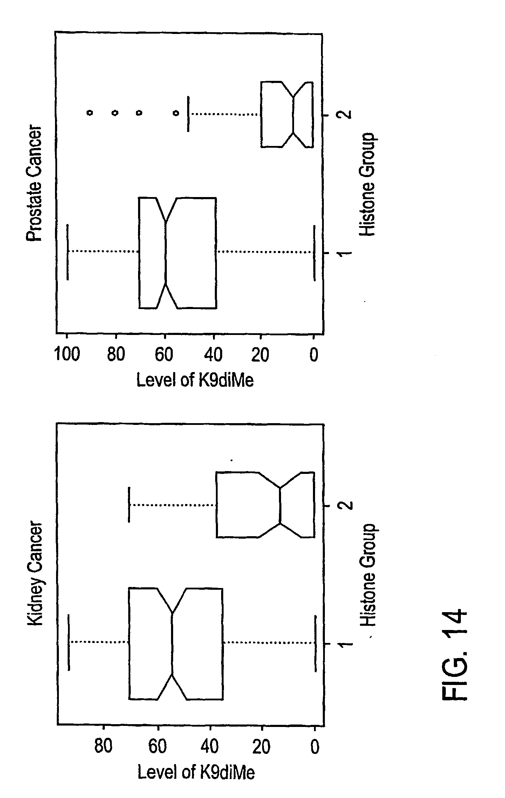

**Figure 14****.** H3 K9diMe is also informative of state of cancer in at least two cancers of prostate and kidney. The percentage of cancer cells that stain positively by immunohistochemistry with an antibody against H3 K9diMe in primary cancer tissues obtained from patients is presented. These are the same tissues that were stained previously with other histone modifications. Patients with poor clinical outcome (prognosis) have significantly lower number of cells that stain positively for H3 K9diMe in both prostate (p=6.4x10⁻¹⁴) and kidney (p=7.5x10⁻¹⁰) cancers. Group 2 has poorer outcome when compared to group 1 in both prostate and kidney cancers.

### DETAILED DESCRIPTION OF THE INVENTION

This present invention is based upon our discovery that changes in global levels of individual histone modifications are associated with the presence of cancer and, importantly, are predictive of clinical outcome. Through immunohistochemical staining of primary prostatectomy samples, the percentage of cells that stain for histone acetylation (Ac) and dimethylation (diMe) of five residues in histones H3 and H4 was determined. Grouping of samples with similar patterns of modifications identified two disease sub-types with distinct risks of tumor recurrence among patients with low-grade prostate cancer. These histone modification patterns were predictors of outcome independent of tumor stage, pre-operative prostate-specific antigen (PSA) levels, and capsule invasion. Thus, widespread changes in specific histone modifications represent novel molecular heterogeneity in prostate cancer, and underlie the broad range of clinical behavior displayed by cancer patients. In subsequent work, the markers were further identified to be useful markers with respect to lung cancer, kidney cancer, breast cancer, colon cancer, and other cancers, as well as prostate cancer.

This evidence indicates that changes in bulk or global histone modifications of cancer cells is predictive of clinical outcome. The mechanistic basis of such changes are currently unclear but maybe related to the altered expression and/or global activities of various histone modifying enzymes. In combinations of two or more, these changes proved to be particularly indicative of risk of tumor recurrence in patients, in particular in patients with low-grade prostate cancer. Considering the substantial number of modifications on histones, information on global patterns of other modification sites would help with further classification of all patients including those in the high-grade category. The utility of immunohistochemistry combined with availability of extensive set of antibodies to probe histone modifications, should facilitate the application of this approach to other tumors and other histone modification patterns. Patterns of histone modifications including, but not limited to those reported here, will serve as prognostic or diagnostic markers in other types of cancer as well. Example 7 which investigated the global H3 K9 dimethylation pattern is in accord with this prediction.

Accordingly, in one aspect, the invention provides a method of diagnosing a cancer by contacting a test tissue sample from an individual at risk of having a cancer or suspected of having cancer with an antibody that specifically binds to a modified histone protein; and determining the global H3K4 dimethylation and H3K18 acetylation histone modification pattern in the test tissue sample in comparison to a control tissue sample; thereby diagnosing said cancer by identification of an altered global histone modification pattern. In some embodiments, the tissue sample is a tumor biopsy sample. In some embodiments, the cancer or tumor is prostate, bladder, kidney, colon or breast cancer. In preferred embodiments, the individual has less advanced disease (i.e. low grade or stage), the category in which diagnostic markers are acutely needed. The global histone modification pattern can be scored according to standard immunohistochemical methodologies.

In another aspect, the invention provides a method of predicting the likelihood of a cancer recurrence based upon an altered global H3K4 dimethylation and H3K18 acetylation histone modification pattern in a tissue sample from the patient taken before, during, or after surgical removal of the cancerous tissue (e.g., prostectomy) or before, during, or after another cancer treatment. The altered global histone modification pattern can be determined as described herein. The cancer can be, for instance, a prostate cancer, ovarian cancer, renal cancer, lung cancer, breast cancer, colon cancer, leukemia, non-Hodgkin's lymphoma, multiple myeloma or hepatocarcinoma. In a preferred embodiment, the cancer is a prostate or bladder cancer. Patients identified as having altered global histone modification pattem(s) associated with an increased risk of metastasis, recurrence or a therapy resistant cancer can be further selected on that basis for treatment with exogenous or endogenous hormone ablation, optionally supplemented with chemotherapy and/or radiation. In the case of prostate cancer, the hormone ablation is androgen ablation (e.g., treatment with finasteride and other anti-tesosterone or anti-DHT agents).

In another aspect, the invention provides a method of providing a prognosis for a cancer by contacting a test tissue sample from an individual at risk for or known to have a cancer with an antibody that specifically binds to a modified histone protein; and determining the global H3K4 dimethylation and H3K18 acetylation histone modification pattern in the test tissue sample in comparison to a control tissue sample; thereby providing a prognosis for said cancer by identification of an altered global histone modification pattern. In some embodiments, the tissue sample is a tumor biopsy sample. In some embodiments, the cancer or tumor is prostate, bladder, kidney, colon or breast cancer. In preferred embodiments, the individual has less advanced disease (i.e. low grade or stage), the category in which prognostic markers are acutely needed.

In another aspect, the invention provides kits comprising at least two antibodies which each bind a different histone protein modification, wherein the antibodies bind the H3K4 dimethylation and H3K18 acetylation histine protein modifications. In some embodiments, the antibodies are labeled with a detectable moiety. In some embodiments, the kits provide reagents for detecting the antibody when bound to a histone protein having the histone protein modification recognized by the antibody. The kits have instructions relating altered histone modification patterns to an increased or decreased risk of cancer metastasis or progression. In other embodiments, the kits further comprise reagents for use in immunohistochemical methods using the antibodies.

In further embodiments of any of the above aspects, the cancer or tumor is prostate, bladder, kidney, colon or breast cancer. The cancer can be a metastatic cancer. The histone proteins and individual are preferably human.

In still another aspect, the invention provides a method of identifying a global histone modification pattern for the diagnosis or prognosis of cancer, said method comprising (a) contacting a test tissue sample from an individual having the cancer with an antibody that specifically binds to a modified histone protein; (b) contacting a control tissue sample from an individual not known to have the cancer with an antibody that specifically binds to the modified histone protein; and (c) determining the global H3K4 dimethylation and H3K18 acetylation histone modification pattern of the modified histone protein in the test tissue sample in comparison to the control tissue sample; thereby identifying an altered global histone modification pattern useful in the diagnosis or prognosis of cancer. The antibody may be a monoclonal antibody or a polyclonal antibody directed toward the histone modification pattern of interest. In some embodiments, the method further comprises the step of fixing the cells and detecting the global histone modifications in the fixed cells.

In further such embodiments, and in any of the above aspects generally, the immunohistochemical staining uses antibodies to specifically bind the histone protein modification of interest. The antibody may be a monoclonal antibody or a polyclonal antibody directed toward the histone modification pattern of interest. The antibody may be labeled with a detectable label (e.g., a radioactive label, and enzymatic label, a fluorescent label, or chemiluminescent label, or a molecular tag). The label bound to the histone modification of interest may be detected by autoradiography, fluorimetry, luminometry, or phosphoimge analysis. In a preferred embodiment, the global histone modifications are detected for a plurality of individual fixed cells in the sample and the intensity and/or frequency of immunohistochemical staining is determined for each of the plurality such that a frequency distribution of cells according to staining intensities are obtained over an area of interest. Preferably, the area of interest focuses on cells having an altered phenotype suggestive of a cancer. The area may be defined empirically according to the region of the sample having the most intense staining (if a modification positively correlates with the risk, grade, or progression of cancer) or the least intense staining if the modification negatively correlates with the risk, grade, or progression of cancer. The area may be of a predetermined size sufficient to provide a valid measure of staining patterns in the area of interest. Multiple areas may be sampled and compared from each of the tissue samples.

In some embodiments, the altered histone modification patterns provide a relative risk for an increased likelihood of a more severe outcome or grade of cancer or of metastasis on the order of at least 1.5, 2, 3, 4, or 5-fold or more or on the order of from 1.5 to 3-fold, or 1.5 to 4-fold, or 2 to 5-fold.

The methods of the invention use antibodies which specifically bind to the histone protein modification of interest to detect the modifications. The antibody may be a monoclonal antibody or a polyclonal antibody directed toward the histone modification pattern of interest. In some embodiments, a plurality of global histone modification patterns are determined for the sample. The histones to be analyzed for particular modifications may be first isolated from the sample and detected using immunochemical methods in a fluid medium.

In some embodiments, the ratio of a modified histone protein to the total levels of the histone protein provide a predictive measure based upon altered global histone modification patterns. For instance, a sample may be analyzed using an antibody which detects modified and unmodified forms of a histone protein and an antibody which selectively detects histones have the modification of interest. The ratio of the two in a population of cells or in a sample is determined and is compared to the ratio for a normal cell to establish a predictive ratio of altered global histone protein modification which can be used in the methods according to the invention.

In some embodiments, the altered global histone modification patterns are predictive of whether a cancer or tumor will be refractory to treatment or therapy resistant.

In some embodiments of any of the above, a histone rule is applied wherein cancer patients having a K4 diMe staining value at or above about the 60 percentile and patients have a better prognosis than patients who are below these levels. In some other embodiments, cancer patients having a K18 Ac and K4 diMe staining value which are each at or above about the 35 percentile have a better prognosis than patients who are below these levels.

In some embodiments of any of the above, the tissue is blood and the altered global histone modification pattern for blood cells is determined. In some embodiments, the samples are from patients who have a leukemia or lymphoma and the altered global histone modification pattern includes patterns from leukemic or lymphoma cells. The detection of the global histone modification patterns can be conducted using immunofuorescence staining of the cells followed by FACS sorting and/or scoring and counting of the cells. These methods can provide a frequency distribution of the global histone modification frequencies for the cells of interest in the sample. In such methods it can also be useful to employ other fluorescent markers identifying the particular cell or its phenotype to facilitate in the sorting and counting of the leukemic or lymphoma cells.

In some embodiments of any of the above, the tissue is disaggregated by enzymatic, grinding, or other means and the global histone modification patterns of individual cells are characterized by immunofluorescence staining using the antibodies described herein followed by FACS sorting and/or scoring and counting of the cells which can provide a frequency distribution of the global histone modification frequencies for the sample. In such methods it can also be useful to employ other fluorescent markers identifying the particular cell or its phenotype to facilitate in the sorting and counting of the particular cells of interest.

### Definitions

Unless otherwise stated, the following terms used in the specification and claims have the meanings given below.

It is noted here that as used in this specification and the appended claims, the singular forms "a," "an," and "the" include plural reference unless the context clearly dictates otherwise.

Amino acids may be referred to herein by either their commonly known three letter symbols or by the one-letter symbols recommended by the IUPAC-IUB Biochemical Nomenclature Commission.

"Global histone modification" refers to patterns of histone protein modification that are not confined to promoter regions but that encompass large areas of chromatin, including non-promoter regions.

"Histone" refers to DNA binding structural proteins of chromosomes. Histones have a high proportion of positively charged amino acids such as lysine and arginine, which aids in DNA binding. The five main types of histones fall into two groups: nucleosomal histones H2A, H2B, H3, H4; and H1 histones. "Modified histone protein" refers to a histone protein with one or more of the following chemical modifications, which include but are not limited to lysine acetylation, lysine methylation (mono-, di-, and trimethylation), lysine ubiquitylation, arginine methylation (mono-, di-, symmetric and asymmetric methylation), serine/threonine/tyrosine phosphorylation.

With regard to amino acid sequence, histones include the proteins of SEQ ID NO:1, SEQ ID NO:2, SEQ ID NO:3, SEQ ID NO:4, SEQ ID NO:5, SEQ ID NO:6 and the naturally occurring variants, including but not limited to the modified histone proteins thereof, as well as proteins which are substantially identical thereto, and in particular, also lack the N-terminal methionine residue at position 1 of the above sequences (e.g., a post-translational loss of the N-terminal methionine residue). Modified histone proteins are well known in the art. For instance, the suitable histone protein modifications may include, but are not limited to, any one or more listed below. Exemplary protein modifications for possible use according to the invention are set forth below.

**Table 1. Histone Protein H4 with potential sites of modification Swiss Prot Acc No: P62805**

| **Pos.** | **AA** | **Mod.** |
|---|---|---|
| 1 | S | P |
| 3 | R | Me |
| 5 | K | Ac |
| 8 | K | Ac |
| 12 | K | Ac Me |
| 16 | K | Ac |
| 20 | K | Ac Me |
| 47 | S | P |
| 59 | K | Me |
| 77 | K | Ac |
| 79 | K | Ac Me |
| 92 | R | Me |

**Table 2. Histone Protein H3 with potential sites of modification Swiss Prot Acc No: Q93081**

| **Pos.** | **AA** | **Mod.** |
|---|---|---|
| 2 | R | Me |
| 3 | T | P |
| 4 | K | Me |
| 9 | K | Ac Me |
| 10 | S | P |
| 11 | T | P |
| 14 | K | Ac Me |
| 17 | R | Me |
| 18 | K | Ac |
| 23 | K | Ac Me |
| 26 | R | Me |
| 27 | K | Ac Me |
| 28 | S | P |
| 32 | T | P |
| 36 | K | Me |
| 37 | K | Me |
| 56 | K | Me |
| 79 | K | Me |
| 115 | K | Ac |
| 118 | T | P |
| 122 | K | Ac |
| 128 | R | Me |

**Table 3: Histone Protein H2A with potential sites of modification SWISS PROT ACC NO:P20671**

| **Pos.** | **AA** | **Mod.** | |
|---|---|---|---|
| 1 | S | P | |
| 5 | K | Ac | |
| 9 | K | Ac | |
| 13 | K | Ac | |
| 15 | K | Ac | |
| 36 | K | Ac | |
| 95 | K | Me | |
| 99 | K | Me | |
| 119 | K | Ac | Ub |
| 120 | T | P | |

**Table 4. Histone Protein H2B with potential sites of modification Swiss Prot Acc No:P62807**

| **Pos.** | **AA** | **Mod.** |
|---|---|---|
| 5 | K | Ac Me |
| 12 | K | Ac |
| 14 | S | P |
| 15 | K | Ac |
| 20 | K | Ac |
| 23 | K | Me |
| 24 | K | Ac |
| 32 | S | P |
| 36 | S | P |
| 43 | K | Me |
| 85 | K | Ac |
| 99 | R | Me |
| 108 | K | Ac |
| 116 | K | Ac |
| 120 | K | Ac Ub |

**Table 5. Selected Histone Modifications**

| | | | **Single Mod** | | **Double Mod** | | **Triple Mod** | |
|---|---|---|---|---|---|---|---|---|
| **Histone** | **Site** | **Modification** | ***mAb*** | ***pAb*** | ***mAb*** | ***pAb*** | ***mAb*** | ***pAb*** |
| **H3** | | un-modified | X | | | | | |
| **14-494** | non-spec | Acetyl | | X | | | | |
| | K4 | Methyl | | X | X | X | | X |
| | K9 | Methyl | | X | X | X | | |
| | K9 | Acetyl | | X | | | | |
| | S10 | Phos | X | X | | | | |
| | K14 | Acetyl | | X | | | | |
| | R17 | Methyl | | | | X | | |
| | K18 | Acetyl | | X | | | | |
| | K23 | Acetyl | | X | | | | |
| | R26 | Methyl | | | | X | | |
| | K27 | Acetyl | | X | | | | |
| | K27 | Methyl | | | | X | | |
| | S28 | Phos | | X | | | | |
| | K36 | Methyl | | | | X | | |
| | K79 | Methyl | | | | X | | |
| | S10/K14 | Phos/Acetyl | | X | | | | |
| | K4/K9 | dimethyl | | | | X | | |
| **H4** | | un-modified | | X | | | | |
| **14-412** | | Hyperacetylated | | X | | | | |
| | | Acetyl | | X | | | | |
| | R3 | Methyl | | | | X | | |
| | K5 | Acetyl | | X | | | | |
| | K8 | Acetyl | | X | | | | |
| | K12 | Acetyl | | X | | | | |
| | K16 | Acetyl | | X | | | | |
| | K20 | Methyl | | X | X | X | | X |
| | K79 | Methyl | | | | X | | |
| | | | | | | | | |
| **H2A** | acidic patch | | | X | | | | |
| **14-493** | non-spec | Ub | X | | | | | |
| | non-spec | Acetyl | | X | | | | |
| | K5 | Acetyl | | X | | | | |
| | K9 | Acetyl | | X | | | | |
| | | | | | | | | |
| **H2B** | | un-modified | | X | | | | |
| **14-491** | non-spec | Acetyl | | X | | | | |
| | K5 | Acetyl | | X | | | | |
| | K12 | Acetyl | | X | | | | |
| | S14 | Phos | | X | | | | |
| | K15 | Acetyl | | X | | | | |
| | K20 | Acetyl | | X | | | | |

In the above tables, "Me" refers to methyl modifications, "Ac" to acetyl modifications, "P" or "phos" refer to phosphorylation modifications, and "Ub" to ubiquinylations. Where Me is indicated it may be a mono-, di, or trimethylation. Where two modifications are listed for a particular protein residue, they can be alternative modifications.

The residue position of tables 1 to 5 is with respect to the positions of SEQ ID Nos: 1 to 6, renumbered without the N-terminal methionine (i.e, the residue position of SEQ ID NOs: 1 to 6 minus one). In preferred embodiments, the histone proteins of SEQ ID NOs:1 to 6 to be detected lack an N-terminal methionine residue.

In another aspect, the invention provides methods of identifying altered global histone modification patterns useful in the diagnosis or prognosis of cancer comprising selecting a global histone modification and comparing the level of the global H3K4 dimethylation and H3K18 acetylation histone modification in samples from cancer patients having different grades or outcomes for cancer and statistically assessing the association of one or more such global histone modifications with the grade or outcome. In some embodiments, the histone modifications are those of any one of Tables 1 to 5.

Antibodies which specifically recognize such variants are well known in the art and often readily commercially available from various sources (*see*, Abcam plc, Upstate Cell Signaling Solutions).

"Immunohistochemistry" refers to the use of antibodies to detect proteins in biological samples such as cells and tissue sections. The detection methods of the present invention can be carried out, for example, using standard immunohistochemical techniques known in the art (*reviewed in* Gosling, Immunoassays: A Practical Approach, 2000, Oxford University Press). Detection is accomplished by labeling a primary antibody or a secondary antibody with, for example, a radioactive isotope, a fluorescent label, an enzyme or any other detectable label known in the art. Visual grading of tissue sections by intensity of staining is well known in the art. Standard controls from tumor and healthy tissue samples are routinely used by those of skill in the art to control for variation among samples and reagents. Moreover, negative controls that do not include primary antibodies specific for the desired target (i.e., histone) are used routinely as controls. Van Diest et al., Anal. Quant. Cytol. Histol. 18(5):351-4 (1996), discloses that even inexperienced observers can, with a few minutes' training, reproducibly grade breast tumor sections on a 0-4 scale based on immunohistochemical staining intensity. Thus, those of skill in the art can grade samples based on a scale (e.g., 0-4 or other), based on percent staining, or based on a simple determination of positive or negative. Methods of immunohistochemical staining are exemplified in the specification. In some embodiments, the frequencies of tissue samples in which an indicated percent or degree of cell staining occurs are ascertained for each modification. Those of ordinary skill in the art appreciate the use of standard controls from tumor and healthy tissue samples to control for variation among samples and reagents. Moreover, negative controls that do not include primary antibodies specific for the desired target can be used routinely to control for background at the time the application was filed. Methods of immunohistochemical scoring are also well known in the art. In some embodiments, immunohistochemical scoring is on a scale from 0 to 4 or 1 to 4. For example, Van Diest et al., Anal. Quant. Cytol. Histol. 18(5):351-4 (1996) disclose that even inexperienced observers can, with a few minutes' training, reproducibly grade breast tumor sections on a 0-4 scale based on immunohistochemical staining intensity."

A "label" or a "detectable moiety" is a composition detectable by spectroscopic, photochemical, biochemical, immunochemical, chemical, or other physical means. For example, useful labels include ³²P, fluorescent dyes, electron-dense reagents, enzymes (e.g., as commonly used in an ELISA), biotin, digoxigenin, or haptens and proteins which can be made detectable, e.g., by incorporating a radiolabel into the peptide or used to detect antibodies specifically reactive with the peptide. Labels may be conjugated directly to the biorecognition molecules, or to probes that bind these molecules, using conventional methods that are well known in the arts. Multiple labeling schemes are known in the art and permit a plurality of binding assays to be performed simultaneously. Different labels may be radioactive, enzymatic, chemiluminescent, fluorescent, quantum dot, or others. Methods of covalently or noncovalently conjugating labels to antibodies are well known to one of ordinary skill in the art. Methods of detecting proteins and modified proteins by use of labeled antibodies are also well known to persons of ordinary skill in the art.

"Cancer" refers to human cancers and carcinomas, sarcomas, adenocarcinomas, lymphomas, leukemias, etc., including but not limited to solid tumors and lymphoid cancers, kidney, breast, lung, kidney, bladder, colon, ovarian, prostate, pancreas, stomach, brain, head and neck, skin, uterine, testicular, esophagus, and liver cancer, lymphoma, including but not limited to non-Hodgkins and Hodgkins lymphoma, leukemia, and multiple myeloma.

"Therapy resistant" cancers, tumor cells, and tumors refers to cancers that have become resistant or refractory to therapy including, but not limited to, aggressive therapy which may be either or both apoptosis-mediated (*e.g*., through death receptor cell signaling, for example, Fas ligand receptor, TRAIL receptors, TNF-R1, chemotherapeutic drugs, radiation) and non-apoptosis mediated (*e.g*., toxic drugs, chemicals) cancer therapies, including, but not limited to, chemotherapy, hormonal therapy, radiotherapy, and immunotherapy.

"Biological sample" includes sections of tissues such as biopsy and autopsy samples, and frozen sections taken for histologic purposes. Such samples include blood and blood fractions or products (e.g., serum, plasma, platelets, red blood cells, and the like), sputum, tissue, cultured cells, e.g., primary cultures, explants, and transformed cells, stool, urine, etc. In one embodiment, the biological sample is a tissue sample prepared for immunohistochemistry. In another embodiment, the biological sample is a tissue sample prepared as a tissue microarray (TMA) for high throughput screening. A biological sample is typically obtained from a eukaryotic organism, most preferably a mammal such as a primate e.g., chimpanzee or human; cow; dog; cat; a rodent, e.g., guinea pig, rat, Mouse; rabbit; or a bird; reptile; or fish.

A "biopsy" refers to the process of removing a tissue sample for diagnostic or prognostic evaluation, and to the tissue specimen itself. Any biopsy technique known in the art can be applied to the diagnostic and prognostic methods of the present invention. The biopsy technique applied will depend on the tissue type to be evaluated (*i.e.,* prostate, lymph node, liver, bone marrow, blood cell), the size and type of the tumor (*i.e*., solid or suspended (*i.e.,* blood or ascites)), among other factors. Representative biopsy techniques include excisional biopsy, incisional biopsy, needle biopsy, surgical biopsy, and bone marrow biopsy. An "excisional biopsy" refers to the removal of an entire tumor mass with a small margin of normal tissue surrounding it. An "incisional biopsy" refers to the removal of a wedge of tissue that includes a cross-sectional diameter of the tumor. A diagnosis or prognosis made by endoscopy or fluoroscopy can require a "core-needle biopsy" of the tumor mass, or a "fine-needle aspiration biopsy" which generally obtains a suspension of cells from within the tumor mass. Biopsy techniques are discussed, for example, in Harrison's Principles of Internal Medicine, Kasper, et al., eds., 16th ed., 2005, Chapter 70, and throughout Part V.

"Antibody" refers to a polypeptide comprising a framework region from an immunoglobulin gene or fragments thereof that specifically binds and recognizes an antigen. The recognized immunoglobulin genes include the kappa, lambda, alpha, gamma, delta, epsilon, and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. Typically, the antigen-binding region of an antibody will be most critical in specificity and affinity of binding.

An exemplary immunoglobulin (antibody) structural unit comprises a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (V_{L}) and variable heavy chain (V_{H}) refer to these light and heavy chains respectively.

Antibodies exist, e.g., as intact immunoglobulins or as a number of well-characterized fragments produced by digestion with various peptidases. Thus, for example, pepsin digests an antibody below the disulfide linkages in the hinge region to produce F(ab)'₂, a dimer of Fab which itself is a light chain joined to V_{H}-C_{H}1 by a disulfide bond. The F(ab)'₂ may be reduced under mild conditions to break the disulfide linkage in the hinge region, thereby converting the F(ab)'₂ dimer into an Fab' monomer. The Fab' monomer is essentially Fab with part of the hinge region (*see* Fundamental Immunology (Paul ed., 3d ed. 1993). While various antibody fragments are defined in terms of the digestion of an intact antibody, one of skill will appreciate that such fragments may be synthesized *de novo* either chemically or by using recombinant DNA methodology. Thus, the term antibody, as used herein, also includes antibody fragments either produced by the modification of whole antibodies, or those synthesized *de novo* using recombinant DNA methodologies (e.g., single chain Fv) or those identified using phage display libraries (*see, e.g.,* McCafferty et al., Nature 348:552-554 (1990))

For preparation of antibodies, e.g., recombinant, monoclonal, or polyclonal antibodies, many techniques known in the art can be used (*see, e.g.,* Kohler & Milstein, Nature 256:495-497 (1975); Kozbor et al., Immunology Today 4: 72 (1983); Cole et al., pp. 77-96 in Monoclonal Antibodies and Cancer Therapy, Alan R Liss, Inc. (1985); Coligan, Current Protocols in Immunology (1991); Harlow & Lane, Antibodies, A Laboratory Manual (1988); and Goding, Monoclonal Antibodies: Principles and Practice (2d ed. 1986)). The genes encoding the heavy and light chains of an antibody of interest can be cloned from a cell, e.g., the genes encoding a monoclonal antibody can be cloned from a hybridoma and used to produce a recombinant monoclonal antibody. Gene libraries encoding heavy and light chains of monoclonal antibodies can also be made from hybridoma or plasma cells. Random combinations of the heavy and light chain gene products generate a large pool of antibodies with different antigenic specificity (*see, e.g.,* Kuby, Immunology (3rd ed. 1997)). Techniques for the production of single chain antibodies or recombinant antibodies (U.S. Patent 4,946,778, U.S. Patent No. 4,816,567) can be adapted to produce antibodies to polypeptides of this invention. Also, transgenic mice, or other organisms such as other mammals, may be used to express humanized or human antibodies (*see, e.g.,* U.S. Patent Nos. 5,545,807; 5,545,806; 5,569,825; 5,625,126; 5,633,425; 5,661,016, Marks et al., Bio/Technology 10:779-783 (1992); Lonberg et al., Nature 368:856-859 (1994); Morrison, Nature 368:812-13 (1994); Fishwild et al., Nature Biotechnology 14:845-51 (1996); Neuberger, Nature Biotechnology 14:826 (1996); and Lonberg & Huszar, Intern. Rev. Immunol. 13:65-93 (1995)). Alternatively, phage display technology can be used to identify antibodies and heteromeric Fab fragments that specifically bind to selected antigens (*see, e.g.,* McCafferty et al., Nature 348:552-554 (1990); Marks et al., Biotechnology 10:779-783 (1992)). Antibodies can also be made bispecific, i.e., able to recognize two different antigens (*see, e.g.,* WO 93/08829, Traunecker et al., EMBO J. 10:3655-3659 (1991); and Suresh et al., Methods in Enzymology 121:210 (1986)). Antibodies can also be heteroconjugates, e.g., two covalently joined antibodies, or immunotoxins (*see, e.g.,* U.S. Patent No. 4,676,980 , WO 91/00360; WO 92/200373; and EP 03089).

Methods for humanizing or primatizing non-human antibodies are well known in the art. Generally, a humanized antibody has one or more amino acid residues introduced into it from a source which is non-human. These non-human amino acid residues are often referred to as import residues, which are typically taken from an import variable domain. Humanization can be essentially performed following the method of Winter and co-workers (*see, e.g.,* Jones et al., Nature 321:522-525 (1986); Riechmann et al., Nature 332:323-327 (1988); Verhoeyen et al., Science 239:1534-1536 (1988) and Presta, Curr. Op. Struct. Biol. 2:593-596 (1992)), by substituting rodent CDRs or CDR sequences for the corresponding sequences of a human antibody. Accordingly, such humanized antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

A "chimeric antibody" is an antibody molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor, drug, etc.; or (b) the variable region, or a portion thereof, is altered, replaced or exchanged with a variable region having a different or altered antigen specificity.

In one embodiment, the antibody is conjugated to an "effector" moiety. The effector moiety can be any number of molecules, including, but not limited to, labeling moieties such as radioactive labels or fluorescent labels, or can be a therapeutic moiety. In one aspect the antibody modulates the activity of the protein.

The phrase "specifically (or selectively) binds" to an antibody or "specifically (or selectively) immunoreactive with," when referring to a protein or peptide, refers to a binding reaction that is determinative of the presence of the protein, often in a heterogeneous population of proteins and other biologics. Thus, under designated immunoassay conditions, the specified antibodies bind to a particular protein at least two times the background and more typically more than 10 to 100 times background. Specific binding to an antibody under such conditions requires an antibody that is selected for its specificity for a particular protein. For example, polyclonal antibodies can be selected to obtain only those polyclonal antibodies that are specifically immunoreactive with the selected antigen and not with other proteins. This selection may be achieved by subtracting out antibodies that cross-react with other molecules. A variety of immunoassay formats may be used to select antibodies specifically immunoreactive with a particular protein. For example, solid-phase ELISA immunoassays are routinely used to select antibodies specifically immunoreactive with a protein (*see, e.g.,* Harlow & Lane, Using Antibodies, A Laboratory Manual (1998) for a description of immunoassay formats and conditions that can be used to determine specific immunoreactivity).

The terms "identical" or percent "identity," in the context of two or more nucleic acids or polypeptide sequences, refer to two or more sequences or subsequences that are the same or have a specified percentage of amino acid residues or nucleotides that are the same (i.e., about 60% identity, preferably 65%, 70%, 75%, 80%, 85%, 90%, 91%, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters described below, or by manual alignment and visual inspection (*see, e.g.,* NCBI web site http://www.ncbi.nlm.nih.gov/BLAST/ or the like). Such sequences are then said to be "substantially identical." This definition also refers to, or may be applied to, the compliment of a test sequence. The definition also includes sequences that have deletions and/or additions, as well as those that have substitutions. As described below, the preferred algorithms can account for gaps and the like. Preferably, identity exists over a region that is at least about 25 amino acids or nucleotides in length, or more preferably over a region that is 50-100 amino acids or nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Preferably, default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well-known in the art. Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (*see, e.g., Current Protocols in Molecular Biology* (Ausubel *et al.,* eds. 1995 supplement)).

A preferred example of algorithm that is suitable for determining percent sequence identity and sequence similarity are the BLAST and BLAST 2.0 algorithms, which are described in Altschul et al., Nuc. Acids Res. 25:3389-3402 (1977) and Altschul et al., J. Mol. Biol. 215:403-410 (1990), respectively. BLAST and BLAST 2.0 are used, with the parameters described herein, to determine percent sequence identity for the nucleic acids and proteins of the invention. Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra*). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4 and a comparison of both strands. For amino acid sequences, the BLASTP program uses as defaults a wordlength of 3, and expectation (E) of 10, and the BLOSUM62 scoring matrix (*see* Henikoff & Henikoff, Proc. Natl. Acad Sci. USA 89:10915 (1989)) alignments (B) of 50, expectation (E) of 10, M=5, N=-4, and a comparison of both strands.

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, γ-carboxyglutamate, and O-phosphoserine. Amino acid analogs refers to compounds that have the same basic chemical structure as a naturally occurring amino acid, i.e., an α carbon that is bound to a hydrogen, a carboxyl group, an amino group, and an R group, e.g., homoserine, norleucine, methionine sulfoxide, methionine methyl sulfonium. Such analogs have modified R groups (e.g., norleucine) or modified peptide backbones, but retain the same basic chemical structure as a naturally occurring amino acid. Amino acid mimetics refers to chemical compounds that have a structure that is different from the general chemical structure of an amino acid, but that functions in a manner similar to a naturally occurring amino acid.

### EXAMPLES

The following example is offered to illustrate, but not to limit the claimed invention.

### EXAMPLE 1:

To determine the global levels of individual histone modifications in tissues obtained from patients, immunohistochemistry, a method for detecting the presence of specific antigens in cells, was combined with Tissue Microarrays (TMA), for high throughput analysis of a large number of tissue samples (Kononen, et al., Nat. Med. 4:844-7 (1998)). The levels of acetylated H3 K9, K18 and H4 K12 and di-methylated H4 R3 and H3 K4 were analyzed using highly specific antibodies (Suka, et al., Mol. Cell 8:473-9 (2001)) (Fig. 5), on 183 primary prostate cancer tissues. The level of staining was assessed independently by two pathologists, who were blinded to all clinico-pathological variables. Here, the global level of staining refers to the percentage of cells within each tissue sample that stains positively for a given antibody. For instance, Fig. 1a-d shows representative staining of four tissue samples, two each for H3 K18Ac (Fig. la-b) and H4 R3diMe (Fig. 1c-d) on tissue arrays. The cells with brown nuclei are considered positively stained. The unstained cells may still contain the modifications at certain genomic loci but their levels are below the detection limits, signifying that bulk histone modifications are reduced considerably in these cells. Therefore, immunostaining reveals presence or absence of global histone modifications in primary tissues.

To assess the differences in staining for the five antibodies, the frequencies (y-axis) of tissue samples in which the indicated percent cell staining (x-axis) are observed for each modification (Fig. 1e) were plotted. Acetylation of H3 K9 and KI8 display very similar distributions, with >65% of samples showing 90-100% staining of the tumor cells. In contrast, only 16% of samples show 90-100% staining for H4 K12 with little or no acetylation detected in ∼24% of samples. Di-methylation of H3 K4 and H4 R3 show broader distributions with >60% of samples staining between 20-80% of cells. These data indicate that the levels of histone modifications differ considerably between individual tissues. As discussed below, these differences are important for defining groups of patients with distinct clinical outcomes.

The relationship between levels of the five histone modifications and degree of differentiation (grade) was determined for all samples. Of the five modifications, H3 K18Ac (*r*=0.28, *P* = 9.6x10⁻⁵), H3 K4diMe (*r*=0.22, *P* =3.4x10⁻³), H4 K12Ac (*r*=0.33, *P* =4.8x10⁻⁶), and H4 R3diMe (*r*=0.23, *P* =2.0x10⁻³) were positively correlated with increasing grade but H3 K9Ac (*r*=0.11, *P* =1.4x10⁻¹) shows no significant correlation. Despite the positive correlations with grades, none of the modifications was associated individually with the risk of tumor recurrence (Table 6). Without being limited by theory, the higher levels of staining may be related to the increased proliferative capacity of de-differentiated tumors, which may be associated with increased gene activity. In this regard, H3 K18Ac and H4 R3diMe are two histone modifications associated with gene activity (Kurdistani, et al., Cell 117:721-33 (2004); Rezai-Zadeh, et al., Genes. Dev. 17:1019-29 (2003)).

**Table 6: Univariate Cox analysis of level of individual histone modifications and risk of tumor recurrence.**

| Histone Modification | Hazard ratio | 95% Cl | *P* value |
|---|---|---|---|
| H3 K9Ac | 0.994 | (0.978 - 1.008) | 0.38 |
| H3 KI8Ac | 0.992 | (0.979 - 1.010) | 0.48 |
| H3 K4diMe | 0.993 | (0.978 - 1.005) | 0.21 |
| H4 K12Ac | 0.994 | (0.979 - 1.009) | 0.47 |
| H4 R3diMe | 0.986 | (0.971 - 1.001) | 0.07 |

The relationships described above are between individual modifications and grade for all patient samples. To determine whether unique patterns of histone modifications, involving combinations of the 5 sites, are shared among sub-sets of tissue samples, the Random Forest (RF) clustering algorithm was applied to the data (Breiman, *Classification and regression trees* (Wadsworth International Group, Belmont, Calif., 1984); Breiman, Machine Learning 45:5-32 (2001); Shi, et al., Mod Pathol. 18:547-57 (2005)). RF clustering is an unsupervised classification method which, by generating an ensemble of individual tree predictors, leads to a natural dissimilarity measure between the observations. The result of the RF clustering of all 183 samples are visualized in Fig. 2a as a multidimensional scaling (MDS) plot. In MDS plots, the orientation and unit of axes are arbitrary but increasing distances between data points reflect increasing degree of dissimilarity. Each patient sample is labeled according to its Gleason score. As delineated by the blue line in Fig. 2a, two groups of patients were identified by inspection and are color-coded as red (*n*=70) and black (*n*=113). In Kaplan-Meier (Kaplan, et al., J. Am. Stat. Assoc. 53:457 (1958)) survival analysis, a statistically significant difference in the risk of tumor recurrence between these two groups (Fig. 2b) was not detected.

However, further stratification of patients into high (Gleason score=7-10) and low (Gleason score=2-6) grade patients, revealed two sub-groups within each category of patients with a considerable difference in their risk of tumor recurrence (Fig. 2c; compare red with blue and green with black lines). These results demonstrate that the differences in histone modification patterns may be a better predictor of clinical outcome when patients are first stratified broadly based on grade. Therefore, patients were first stratified into those with Gleason score>7 (*n*=79) or Gleason score<7 (*n*=104; Table 7), and then the RF clustering algorithm was applied. In the high-grade patients, distinct groups based on histone modifications were not clearly evident (data not shown). However, among the low-grade patients, two groups of patients were found by inspection (Fig. 3a). The median levels and distribution of staining for the various modifications in the two groups are shown in the boxplots (Cleveland, Visualizing data (At&T Bell Laboratories; Published by Hobart Press, Murray Hill, N.J., 1993)) of Fig. 3b. As expected from the clustering, the two groups show different modification patterns. For instance, the median percent cell staining for H3 K9Ac in group 1 is 90% whereas that of group 2 is 16%. Interestingly, within each group of patients, different histone modifications show differential levels of staining. For instance, within group 1, the median percent cell staining for H3 K9Ac is 100% whereas that of H4 K12Ac is 68%. Similarly, within group 2, the median percent cell staining for H3 K18Ac is 52% whereas that of H4 K12Ac is 5%. Taken together, these observations indicate that groups of patients can be identified based on similar combinations of global histone modifications.

**Table 7. Relationship of Histone Modification Groups with Clinicopathologic Parameters in Low Grade (Gleason Score 2-6) Prostate Adenocarcinomas n=104)**

| | **All Low Grade Patients** | **Group 1 (Percent of Total)^{a}** | **Group 2^{*} (Percent of Totala)** | **P-value^{b}** |
|---|---|---|---|---|
| **Total Cases** (n=104) | | 56 (54) | 48 (46) | |
| **Age At Surgery** | | | | 0.58 (NS)^{c} |
| (n=104) | 64.0 (46.0-75.0) | 64.0 (49.0-75.0) | 64.0 (49.0-75.0) | |
| Median (Range) | | | | |
| **Pathology pT Staged** (n=104) | | | | 0.66 (NS) |
| PT2-pT3a | 99 (95) | 54 (96) | 45 (84) | |
| PT3b | 5 (5) | 2 (4) | 3 (6) | |
| **Lymph Node Status** | | | | >0.99 (NS) |
| (n=102) | | | | |
| Positive | 2 (2) | 1 (2) | 1 (2) | |
| Negative | 100 (98) | 54 (98) | 46 (98) | |
| **Tumor Margins** | | | | 0.36 (NS) |
| n=103 | | | | |
| Positive | 25 (24) | 11 (20) | 14 (29) | |
| Negative | 78 (76) | 44 (80) | 34 (71) | |
| **Capsular Invasion** | | | | 0.15 (NS) |
| (n=104) | | | | |
| No Invasion | 28 (27) | 18 (32) | 10 (21) | |
| Invasion | 56 (54) | 29 (52) | 27 (56) | |
| Extension | 20 (19) | 9 (16) | 11 (23) | |
| **Organ Confined^{f}** | | | | 0.70 (NS) |
| (n=104) | | | | |
| Yes | 67 (64) | 40 (71) | 27 (56) | |
| No | 3 (36) | 16 (29) | 21 (44) | |
| **High Risk⁸** (n=102) | | | | |
| Yes | 7 (7) | 3 (5) | 4 (9) | |
| No | 95 (93) | 52 (95) | 43 (91) | |
| **PreOpPSA (ng/ml)** | | | | 0.63 (NS)^{c} |
| (n=93) | | | | |
| Median (Range) | 7.8 (0.6-56.0) | 7.6 (0.6-34.0) | 7.9 (2.1-56.0) | |
| Mean | 10.4 | 9.6 | 11.4 | |
| | | | | |
| **Recurrence^{g}** (n=104) | | | | |
| Yes | 20 (19) | 6 (11) | 14 (29) | |
| No | 83 (81) | 49 (89) | 34 (71) | |
| Total Follow-up', | | | | 0.44^{c} |
| months | | | | |
| Median (Range) | 60.0 (2.0-163.0) | 65.0 (2.0-163.0) | 48.0 (2.0-151.0) | |
| Mean | 61.0 | 67.7 | 53.0 | |
| Total Follow-up, in Recurred group | | | | |
| (n=20) | 30.5 (2.0-98.0) | 32.0 (29.0-75.0) | 12.5 (2.0-98.0) | |
| Median (Range) | 31.6 | 41.3 | 27.4 | |
| Mean | | | | |
| Total Follow-up in | | | | |
| Non-Recurring group | | | | |
| (n=83) | | | | |
| Median (Range) | 65.5 (2.0-163.0) | 71.0 (2.0-163.0) | 63.0 (6.0-151.0) | |
| Mean | 68.5 | 71.1 | 64.5 | |

| | | | | |
|---|---|---|---|---|
| ^{a} Row percentage totals are shown for 'Total Cases'; in all other categories column percentage totals are shown ^{b} P-value was determined by the Fisher's Exact test unless otherwise specified ^{c} P-value was determined by the Mann-Whitney test ^{d} pT3b indicates seminal vesicle invasion. There are no pT4 cases ^{e} P-value was determined by the Pearson's Chi Square test. ^{f} Organ Confined = no capsular extension nor seminal vesicle invasion nor lymph node involvement, and margins are negative ^{g} High Risk for recurrence due to seminal vesicle invasion and/or nodal metastases ^{h} Recurrence = PSA elevation rising >0.2 ng/ml status post radical prostatectomy ⁱ Follow-up = time to recurrence or last follow-up * According to the "simple rule", 2 members of Group 2 fall into Group 1. | | | | |

To ascertain whether the identified groups are clinically significant,the risk of tumor recurrence in each group was determined after removal of primary tumor (Fig. 4a). Remarkably, the patients in group 1 exhibited a lower risk of 10-year tumor recurrence (17%) when compared to those in group 2 (42%) (*P*=0.0076). Importantly, grade does not substitute for the histone modifications as there is no significant difference in the distribution of patients based on the Gleason score between the two groups (Fisher's Exact test, *P*>0.2; Fig. 4a). With regard to whether the modification patterns add prognostic information beyond other known prognostics factors, the histone modification patterns predict tumor recurrence independent of tumor stage, pre-operative PSA, and capsule invasion (Table 8). Thus, patterns of global histone modifications represent independent molecular markers associated with distinct clinical outcomes.

**Table 8: Multivariate proportional hazard analysis**

| Variable | Hazard ratio | 95% Cl | P value |
|---|---|---|---|
| Tumor Stage | 7.54 | (1.86 - 30.47) | 0.0046 |
| Preoperative serum PSA (ng/ml) | 1.02 | (0.98 - 1.07) | 0.3100 |
| Capsule Invasion | 3.41 | (1.40 - 8.30) | 0.0070 |
| Histone Modification Patterns | 3.86 | (1.18 - 12.62) | 0.0250 |

While all five modification sites contributed to the grouping of patients above, the identified groups can also be estimated using less information. For instance, group 1 individuals with lower risk of tumor recurrence can be identified as those patients who are above 60 percentile staining for H3 K4diMe or above 35 percentile staining for H3 K18Ac and above 35 percentile staining for H3 K4diMe; those patients that do not satisfy this rule belong to group 2 (Supplementary Fig. S2). When this rule is used to predict recurrence risk, it results in only two misclassified patients (Log rank *P*-value=0.028; hazard ratio=2.8, 95% *CI*=1.12-7.02). Thus, although additional information leads to more significant groupings, simpler rules involving a limited number of modifications can be constructed that may prove to be more practical in clinical settings.

To validate the prognostic power of histone modifications, an additional independent set of 39 patient samples with low grade prostate cancer (obtained from University of Michigan Medical School) was analyzed according to the above simple rule involving H3 K18Ac and K4diMe staining (Table 9). As shown in Fig. 4b, the staining distinguishes two groups of patients with distinct risks of tumor recurrence-4% in group A vs. 31 % in group B (Log rank *P*-value=0.016; hazard ratio=9.2, 95% *CI*=1.02-82.2). There is no significant difference in the distribution of patients based on the Gleason score between the two groups (Fisher's Exact test, *P*>0.2; Fig. 4b). Thus, the prognostic classification on the validation set confirms the predictive power of histone modifications as markers of prognosis.

**Table 9. Relationship of Histone Modification Groups with Clinicopathologic Parameters in Low Grade (Gleason Score 5-6) Prostate Adenocarcinomas from the Michigan Validation TMA (n-39).**

| | **All Low Grade Patients** | **Group A (Percent of Total^{a})** | **Group B (Percent of Total^{a})** | **^{*} P-value^{b}** |
|---|---|---|---|---|
| **Total Cases** (n=39) | | 26 (67) | 13 (33) | |
| **Age At Surgery** | | | | 0.99 (NS)^{c} |
| (n=39) | | | | |
| Median (Range) | 60.0 (50.0-73.0) | 60.0 (50.0-68.0) | 60.0 (50.0-73.0) | |
| **Pathology pT Stage^{d}** (n=39) | | | | (NA) |
| PT2-pT3a | 39 (100) | 26 (100) | 13 (100) | |
| PT3b | 0 (0) | 0 (0) | 0 (0) | |
| **Lymph Node Status** | | | | >0.99 (NS) |
| (n=39) | | | | |
| Positive | 1 (3) | 1 (4) | 0 (0) | |
| Negative | 38 (97) | 25 (96) | 13 (100) | |
| **Tumor Margins** | | | | 0.99 (NS) |
| n=39 | | | | |
| Positive | 7 (18) | 5 (19) | 2 (15) | |
| Negative | 32 (82) | 21 (81) | 11 (85) | |
| **Capsular Invasion** | | | | 0.28 (NS)^{e} |
| (n=39) | | | | |
| No Invasion | 31 (79) | 22 (85) | 9 (69) | |
| Invasion | 7 (18) | 4 (15) | 3 (53) | |
| Extension | 1 (3) | 0 (0) | 1 (8) | |
| **Organ Confined^{f}** | | | | >0.99 (NS) >0.99 (NS) |
| (n=39) | | | | |
| Yes | 25 (64) | 17 (65) | 8 (62) | |
| No | 14 (36) | 9 (35) | 5 (38) | |
| **High Risk^{g}** (n=39) | | | | |
| Yes | 1 (3) | 1 (4) | 0 (0) | |
| No | 38 (97) | 25 (96) | 13 (100) | |
| **PreOpPSA (ng/ml)** | | | | 0.013^{c} |
| (n=39) | | | | |
| Median (Range) | 5.0 (1.2-36.7) | 4.2 (1.2-11.0) | 6.7 (3.3-36.7) | |
| Mean | 6.8 | 4.9 | 10.7 | |
| | | | | |
| **Recurrence^{h}** (n=39) | | | | |
| Yes | 5(13) | 1(4) | 4(31) | |
| No | 34 (87) | 25 (96) | 9 (69) | |
| Total Follow-up^{l}, | | | | 0.37 (NS)^{c} |
| months | | | | |
| Median (Range) | 61.9 (4.2-110.0) | 65.7 (11.5-105.6) | 60.3 (4.2-110.0) | |
| Mean | 61.5 | 65.0 | 54.4 | |
| Total Follow-up, in | | | | |
| Recurred group (n=5) | | | | |
| Median (Range) | 9.2 (4.2-36.9) | 36.9 (36.9-36.9) | 7.8 (4.2-30.8) | |
| Mean | 17.5 | 36.9 | 12.6 | |
| Total Follow-up in | | | | |
| Non-Recurring group | | | | |
| (n=34) | | | | |
| Median (Range) | 69.0 (11.5-110.0) | 67.8 (11.5-105.6) | 70.3 (44.6-110.0) | |
| Mean | 68.0 | 66.1 | 73.0 | |

| | | | | |
|---|---|---|---|---|
| ^{a} Row percentage totals are shown for 'Total Cases'; in all other categories column percentage totals are shown ^{b} P-value was determined by the Fisher's Exact test unless otherwise specified ^{c} P-value was determined by the Mann-Whitney test ^{d} pT3b indicates seminal vesicle invasion. There are no pT4 cases ^{e} P-value was determined by the Pearson's Chi Square test. ^{f} Organ Confined = no capsular extension nor seminal vesicle invasion nor lymph node involvement, and margins are negative ^{g} High Risk for recurrence due to seminal vesicle invasion and/or nodal metastases ^{h} Recurrence = PSA elevation rising >0.2 ng/ml status post radical prostatectomy ⁱ Follow-up = time to recurrence or last follow-up | | | | |

### Methods

### Prostate Tissue Microarray (TMA)

A prostate TMA was constructed using formalin-fixed, paraffin-embedded prostate tissue samples as previously described (Kononen, et al., Nat. Med. 4:844-7 (1998)). At least 3 replicate tumor samples were taken from donor tissue blocks in a highly representative fashion. Twenty patients treated with neoadjuvant hormones were excluded from the study. In total, 183 cases were informative for all 5 histone markers and 171 of those were supported by complete recurrence data. A retrospective analysis for outcome assessment was based on detailed anonymized clinicopathologic information linked to the TMA specimens. Recurrence, defined as a postoperative serum PSA of 0.2 ng/ml or greater, was seen in 61 (34%) of all study patients, and 20 (19%) of patients with low-grade tumors. The median total follow-up, defined as the time to recurrence or to last contact in non-recurring patients, was 60.0 months (range 2-163) for patients with low-grade tumors. The median follow-up time within the recurring and non-recurring patient groups was 30.5 (2.0-98.0) and 65.5 months (range 2.0-163.0), respectively, in low-grade patients. The validation dataset was generated from prostate TMA's that were purchased from the University of Michigan Medical School (Table 9).

### Immunohistochemistry

The antibodies were first tested and optimized on whole tissue sections and test arrays. Once an appropriate dilution was determined, a set of 3 slides containing all patient samples were stained for each antibody, using standard 2-step indirect immunohistochemistry. Tissue array sections were cut immediately prior to staining using a sectioning aid (Instrumedics, NJ). Following deparaffinization in xylenes, the sections were rehydrated in graded alcohols. Endogenous peroxidase was quenched with 3% hydrogen peroxide in methanol at room temperature. The sections were placed in 95°C solution of 0.01 M sodium citrate buffer (pH 6.0) for antigen retrieval. 5% normal goat serum was next applied for 30 min to block non-specific protein binding sites.

Primary rabbit anti-histone polyclonal antibodies were applied for 30 min at room temperature at following dilutions: H3K18Ac at 1:200; H3K9Ac at 1:800; H4K12Ac at 1:100; H3K4diMe (Upstate) at 1:800; and H4R3diMe (Abeam) at 1:25. Detection was accomplished using the DAKO Envision System, followed by chromogen detection with diaminobenzidine (DAB). The sections were counterstained with Harris' Hematoxylin, followed by dehydration and mounting. Negative controls were identical array sections stained minus the primary antibody. Semi-quantitative assessment of antibody staining on the TMAs was performed by persons blinded to all clinicopathologic variables. The frequency of nuclear positive target cells (range 0-100%) in prostatic glandular epithelium was scored for each TMA spot.

### Unsupervised clustering algorithm

To facilitate unsupervised learning, an intrinsic dissimilarity measure between the patients was constructed with a Random Forest analysis of the histone markers. For a technical description of the RF clustering algorithm, consult http://www.genetics.ucla.edu/labs/horvath/RFclustering/RFclustering.htm. The RF clustering algorithm was shown recently to be particularly suitable for TMA data for the following reasons (Shi, et al., Mod. Pathol. 18:547-57 (2005)). First, the clustering results do not change when one or more covariates are monotonically transformed since the dissimilarity only depends on the feature ranks, obviating the need for symmetrizing skewed covariate distributions. Second, the RF dissimilarity weighs the contributions of each covariate on the dissimilarity in a natural way: the more related the covariate is to other covariates the more it will affect the definition of the RF dissimilarity. Third, the RF dissimilarity does not require the user to specify threshold values for dichotomizing tumor expressions. External threshold values for dichotomizing expressions in unsupervised analyses may reduce the information content or even bias the results. The e RF clustering approach was also compared to the standard Euclidean distance-based approach. Although there is good overlap between the two algorithms, the RF clustering method was preferably used. To visualize the clustering, classical multidimensional scaling was used. This scaling takes as input the RF dissimilarity between the samples and returns a set of points in a 2 dimensional space such that the distances between the points are approximately equivalent to the original distances.

### Statistical analysis

To test whether variables differed across groups, the Kruskal-Wallis test was used. To visualize the survival distributions, Kaplan-Meier plots were used. The Cox proportional hazards model was used to test the statistical independence and significance of predictors. The proportional hazard assumption was tested using scaled Schoenfeld residuals. Log-rank tests were used to test the difference between survival distributions. All P-values were two sided and p<0.05 was considered significant. All statistical analyses were carried out with the freely available software R (http://www.R-project.org/. R code that implements RF clustering is available upon request.

### EXAMPLE 2

Considering that histones and their modifications are present ubiquitously, the above results in prostate cancer raised two important questions: Do the histone modification patterns identified in prostate cancer, serve as universal markers of prognosis to predict clinical outcome in other cancer types? Are these patterns pre-existing in normal tissues or acquired during carcinogenesis? The evidence here shows that the level of staining of the same two histone modifications, H3 K4diMe and K18Ac is useful to distinguish patients with distinct clinical outcomes in three different carcinomas of lung, kidney and breast. The developed data show that these predictive patterns are not pre-existing and likely arise during carcinogenesis.

To determine the level of global histone modifications in tissues obtained from patients, immunohistochemistry (IHC), a method for detecting the presence of specific antigens in cells, was combined with Tissue Microarrays (TMA), for high throughput analysis of a large number of tissue samples. The levels of H3 K18Ac and H3 K4diMe were analyzed using antibodies that recognize these specifically modified residues. The level of staining was assessed independently by two pathologists, who were blinded to all clinico-pathological variables. Here, the global level of histone modifications refers to the percentage of cancer cells within each tissue sample that stained positively for a given antibody. Shown in Fig 10a-d is representative staining of cancer tissues from lung (Fig. 10a), kidney (Fig. 10b), and breast (Fig. 10c) as well as matched normal kidney tissue (Fig. 10d) with anti-H3 K18Ac antibody. The cells with brown nuclei are considered positively stained, and their percentage within the tumour tissue is determined. The unstained cells may still contain the modifications at certain genomic loci but their levels are below the detection limits, signifying that bulk histone modifications are considerably decreased in these cells.

To assess the distribution of staining for the two antibodies, the frequencies (y axis) of tissue samples in which the indicated percentage cell staining (x axis) were observed were plotted for each modification in lung (Fig. 10e), kidney (Fig. 10f) and breast (Fig. 10g) specimens. In lung cancer, H3 K4diMe staining shows a broad distribution whereas K18Ac staining is skewed toward higher percent cell staining. In kidney carcinoma, there is a broad distribution of staining levels for either H3 K18Ac or K4diMe with <10% of specimens showing 90-100% staining. In breast cancer, the distributions of staining for both modifications are skewed toward higher levels of staining, with 40-50% of samples showing 80-100% positive staining for either modification. These data indicate that the incidence of H3 K18Ac and K4diMe can differ considerably not only for a single patient's tissue but also between tissues from different individuals. As shown below, these differences define groups of patients with distinct clinical outcomes.

In order to determine whether histone modifications can predict clinical outcome in each cancer type, patients were first stratified into broad clinical categories based on histological findings such as grade or stage, then assigned patients from each category into two groups according to the levels of H3 K18Ac and K4diMe staining. Finally, it was determined whether the two groups have different clinical outcomes. The rationale for the initial broad stratification is that grade and stage are strong predictors of outcome. Grade is a histological measure of tumour differentiation. Stage is a measure of tumour spread beyond its original site. In general, higher grade and stage are associated with poorer outcome. However, since grade and stage stratification are based on histology, each stratum may include sub-types that are molecularly heterogeneous. In the case of prostate cancer, histone modifications were independent predictors of outcome in low-grade patients. Similar to the findings in prostate cancer, the histone modification patterns surprisingly predict outcome in patients with less advanced disease (i.e. low grade or stage), the category in which prognostic markers are acutely needed.

### EXAMPLE 3

To determine whether histone modification patterns are clinically informative in lung cancer, patients were partitioned according to a 'histone rule' that was developed previously from the prostate cancer data. The rule specifies that the patients who are above 60 percentile staining for K4diMe or 35 percentile staining for K18Ac and K4diMe have better prognosis than those who are below these levels. (Percentile reflects the relative standing of a value in a dataset). The 'histone rule' separates the patients with high levels of K4diMe and/or K18Ac from those that have low levels of both. Applying this rule to patients with stage 1 lung adenocarcinomas (n=117), it is found that the patients who satisfy the rule (i.e., high levels of K4diMe and K18Ac) have 15-year survival probability of 71% (black line, Fig. 11a); those who do not satisfy the rule (i.e., low levels of either or both modifications) have a 15-year survival probability of 32% (red line, Fig. 11a) (p=0.0034, Hazard ratio (HR)=2.93, 95% CI=1.38-6.23). Between the two groups, there is no significant difference in grade, tumour size, gender or age at surgery (Fig. 11a inset box and data not shown). In stages 2, 3 and 4, detect subgroups with significant differences in clinical outcome were not detected. Thus, the same prognostic histone modification pattern in prostate cancer, serves as independent marker of prognosis in lung adenocarcinoma.

### EXAMPLE 4

Applying a smilar 'histone rule' as above (i.e., >60 or >35 percentile staining for H3 K4diMe and K18Ac, respectively) to the patients with organ-confined kidney tumours (n=156), two groups of patients were identified which differ significantly in their survival probabilities. The patients who satisfy the rule have a 1-year survival probability of 81 %; those who fall below these levels for both modifications and thus, do not satisfy the rule have a 1-year survival probability of 52% (p=0.012, HR=2.56, 95%CI=1.20-5.48). There is no difference in the distribution of patients according to grade between the two groups. In fact, when the same analysis for patients in a given grade category is done, similar results are obtained. For instance, application of the 'histone rule' to the grade 2 patients only (n=147) identified two sub-types with significantly different survival probabilities, 51% vs. 19% (p=0.0017, HR=2.26, 95%CI=1.34-3.81) (Fig. 2b). Thus, as in prostate and lung cancers, the levels of two histone modifications serve as independent markers of prognosis in kidney carcinomas.

### EXAMPLE 5

High levels of H3 K4diMe and low levels of H3 K18Ac were associated with increased risk of metastasis. The 'histone rule' above divides patients into two groups: those with high levels of one or both modifications and those with low levels of both. To determine whether further partitioning of patients can be clinically informative, the clinical status of patients with high levels of one modification but low levels of the other was examined. These include patients with >60 and <35 percentile H3 K4diMe and K18Ac, respectively (Group K4), and those with <60 and >35 percentile H3 K4diMe and K18Ac, respectively (Group K18). Remarkably, 67% of patients in Group K4, (12 of 18) had metastatic disease at the time of diagnosis. In contrast, 37% of patients (36 of 98) in Group K18 had metastasis at the time of diagnosis (Fisher's Exact test p=0.021) (Fig. 12). Among the patients with high or low levels of both modifications, a significant difference in distribution of patients with metastatic disease was not detected (data not shown). Thus, high levels of H3 K4diMe and low levels of H3 K18Ac are associated with increased risk of metastasis.

### EXAMPLE 6

A histone modification pattern predicts tumour recurrence in breast cancer. To determine whether histone modification patterns are clinically informative in breast cancer, the 'histone rule' (i.e., >60 or >35 percentile staining for H3 K4diMe and K18Ac, respectively) was applied to the patients with grades 1 and 2 breast tumours (n=33). The 'histone rule' identified two groups of patients with significantly different risks of tumour recurrence. The patients who satisfy the rule have <1% risk of 8-year tumour recurrence whereas those who do not, have 30% risk of recurrence (p=0.006, Fig. 11c). Between the two groups, there is no significant difference in grade, estrogen and progesterone receptor expressions, and tumour size (Fig. 2c inset box and data not shown). Thus, as in prostate, lung and kidney cancers, global levels of H3 K4diMe and K18Ac serve as independent markers of prognosis in breast carcinoma.

Predictive histone modifications patterns are not associated with normal tissue and likely arise during carcinogenesis. The tissues examined so far are all from cancer specimens. To determine whether patterns in normal tissue can be predictive of clinical outcome in cancer, the levels of H3 K4diMe and K18Ac in matched normal tissue from kidney cancer patients were examined. The normal tissues were included in the same tissue microarray as the cancer samples and so, were stained concurrently. Figure 10h shows the distribution of staining for the two histone modifications. Similar to the cancer tissues, the normal tissues show considerable heterogeneity in the levels of histone modifications between individuals. However, the levels of the two histone modifications showed low correlations between cancer and matched normal tissues (rK4=0.27 and rK18=0.27), indicating little similarity in the patterns of their occurrences in cancer versus normal samples. Indeed, application of the 'histone rule,' as developed from the cancer data, to the normal data generated two groups with no significant difference in their probabilities of survival (Fig.11d). Further analysis of the normal data did not reveal any predictive pattern (data not shown). Inclusion of normal data with those of cancer did not increase the predictive power of histone modifications (data not shown). Nonetheless, when patients are categorized based on the differences in level of histone modifications between normal and cancer tissues for either K4diMe or K18Ac, those who have less staining in their cancer compared to normal tissues were significantly enriched for patients with poorer survival probability.

Taken together, these results suggest that, despite substantial epigenetic heterogeneity, predictive histone modifications patterns are not associated with normal tissue and likely arise during carcinogenesis. This is strong evidence that similar cellular epigenetic heterogeneity in cancer tissues predict disease outcome in different carcinomas of lung, kidney and breast in addition to the previously reported prostate cancer. This general and independent prognostic power is unlike most markers of prognosis described to date in cancer. In all cancers examined so far, patients who have higher percentage of cancer cells with both H3 K4diMe and K18Ac have better prognosis than those with lower percentages (Fig. 13). Thus, the increased prevalence of malignant cells with no detectable H3 K4diMe and K18Ac by immunohistochemistry is associated with poor outcome regardless of tissue of origin. It remains to be determined whether these cells are derived from a single precursor cell (i.e., clonal) or from gradual loss of histone modifications in different tumour cells within a tissue.

Mechanistically, global loss of histone modifications may be due to inactivation of histone acetyl- and methyltransferases or aberrant activation of histone deacetylases and demethylases. The data suggest that such mechanisms that lead to global loss of H3 K4diMe and K18Ac (and potentially other histone modifications) may be common to carcinomas of different origins. Considering that matched normal tissues in kidney cancer patients lack predictive histone modification patterns, it is likely that loss of H3 K4diMe and K18Ac occur during cancer development and/or progression. Regardless of mechanism, this invention should be an effective assay to identify cancer patients who would particularly benefit from epigenetic therapy.

**Immunohistochemistry.** A standard 2-step indirect immunohistochemical staining method was used for all antibodies. Tissue array sections (4 µm-thick) were cut immediately prior to staining using a sectioning aid (Instrumedics, NJ). Following deparaffinization in xylenes, the sections were rehydrated in graded alcohols. Endogenous peroxidase was quenched with 3% hydrogen peroxide in methanol at room temperature. The sections were placed in 95°C solution of 0.01 M sodium citrate buffer (pH 6.0) for antigen retrieval. 5% normal goat serum was next applied for 30 min to block non-specific protein binding sites. Primary rabbit anti-histone polyclonal antibodies were applied for 30 min at room temperature- H3K18Ac at 1:200 and H3K4diMe (Upstate) at 1:800 dilution from stock. Detection was accomplished using the DAKO Envision System, followed by chromogen detection with diaminobenzidine (DAB). Incubations were performed in a humidity chamber. The sections were counterstained with Harris' Hematoxylin, followed by dehydration and mounting. Negative controls were identical array sections stained minus the primary antibody.

**Scoring of immunohistochemistry.** Semi-quantitative assessment of antibody staining on the TMAs was performed by two pathologists blinded to the clinicopathologic variables. Both tissue spot histology and grading were confirmed on Hematoxylin and Eosin stained TMAs and all the counterstained study slides. The frequency of nuclear positive target cells (range 0-100%) was scored for each TMA spot.

**Statistical analysis.** To test whether variables differed across groups, the Kruskal-Wallis test, a non-parametric multi-group comparison test, was used. To visualize the survival distributions, Kaplan-Meier plots were used. The Cox proportional hazards model was used to test the statistical independence and significance of predictors. The proportional hazard assumption was tested using scaled Schoenfeld residuals. Log-rank tests were used to test the difference between survival distributions. All p-values were two sided and p<0.05 was considered significant. All statistical analyses were carried out with the freely available software R (http://www.r-project.org/).

### EXAMPLE 7

This example provides information that in addition to previously indicated histone modifications [i.e., histone H3 lysine (K)9 acetylation (Ac), H3 K18Ac, H4 K12Ac, H3 K4 dimethylation (diMe), and H4 arginine (R)3diMe], another histone modification, namely, H3 K9diMe is also informative of state of cancer in at least two cancers of prostate and kidney. The percentage of cancer cells that stain positively by immunohistochemistry was determined using an antibody against H3 K9diMe in primary cancer tissues obtained from patients. These are the same tissues that were stained previously with other histone modifications. It was found that that patients with poor clinical outcome (prognosis) have significantly lower number of cells that stain positively for H3 K9diMe in both prostate (p=6.4x10⁻¹⁴) and kidney (p=7.5x10⁻¹⁰) cancers. This result indicates that lower levels of H3 K9diMe is associated with poor prognosis. This is indicated in the figure 14. Group 2 has poorer outcome when compared to group 1 in both prostate and kidney cancers. Therefore, H3 K9diMe also can be used as a marker of prognosis in difference cancers.

### SEQ ID NO:1 histone H3 [Homo sapiens].

histone H3 [Homo sapiens].
   ACCESSION CAB06030
   VERSION CAB06030.1 GI:1749796
Albig,W. and Doenecke,D.; The human histone gene cluster at the D6S105 locus. Hum. Genet. 101 (3), 284-294 (1997)
   PUBMED 9439656
Amino acid sequence including the N-terminal methionine residue:

1 martkqtark stggkaprkq latkaarksa patggvkkph ryrpgtvalr eirryqkste
61 llirklpfqr lvreiaqdfk tdlrfqssav malqeaceay lvglfedtnl caihakrvti
121 mpkdiqlarr irgera

### SEQ ID NO:2 histone 3.3 human

Histone H3.3.
   ACCESSION P84243
   VERSION P84243 GI:55977062
Wells,D. and Kedes,L., Structure of a human histone cDNA: evidence that basally expressed histone genes have intervening sequences and encode polyadenylylated mRNAs. Proc. Natl. Acad. Sci. U.S.A. 82 (9), 2834-2838 (1985)
   PUBMED 2859593
Amino acid sequence including N-terminal methionine:

1 martkqtark stggkaprkq latkaarksa pstggvkkph ryrpgtvalr eirryqkste
61 llirklpfqr lvreiaqdfk tdlrfqsaai galqeaseay lvglfedtnl caihakrvti
121 mpkdiqlarr irgera

### SEQ ID NO:3 histone H4 (homo Sapiens)

histone H4 [Homo sapiens].
   ACCESSION CAA58538
   VERSION CAA58538.1 GI:603556
   AUTHORS Albig,W., Meergans,T. and Doenecke,D. Characterization of the H1.5 gene completes the set of human H1 subtype genes. Gene 184 (2), 141-148 (1997)
   PUBMED 9031620
Amino acid sequence including N-terminal methionine:

1 
61 

### SEQ ID No:4 histone 2A (human)

histone H2A [Homo sapiens].
   ACCESSION NP_778235
   VERSION NP_778235.1 GI:28195394
   Lusic,M., Marcello,A., Cereseto,A. and Giacca,M. Regulation of HIV-1 gene expression by histone acetylation and factor recruitment at the LTR promoter. EMBO J. 22 (24), 6550-6561 (2003)
   PUBMED 14657027
Amino acid sequence including N-terminal methionine:

1 msgrgkqggk arakaksrss raglqfpvgr vhrllrkgny aervgagapv ylaavleylt
61 aeilelagna ardnkktrii prhlqlavrn deelnkllgg vtiaqggvlp niqavllpkk
121 teshkpgknk

### SEQ ID NO:5 Histone H2B

histone H2B [Homo sapiens].
   ACCESSION CAA41051
   VERSION CAA41051.1 GI:510991
   Collart,D., Romain,P.L., Huebner,K., Pockwinse,S., Pilapil,S., Cannizzaro,L.A., Lian,J.B., Croce,C.M., Stein,J.L. and Stein,G.S. A human histone H2B.1 variant gene, located on chromosome 1, utilizes alternative 3' end processing. J. Cell. Biochem. 50 (4), 374-385 (1992)
   PUBMED 1469070
Amino acid sequence including N-terminal methionine:

1 mpepaksapa pkkgskkavt kaqkkdgkkr krsrkesysi yvykvlkqvh pdtgisskam
61 gimnsfvndi feriageasr lahynkrsti tsreiqtavr lllpgelakh avsegtkavt
121 kytssk

### SEQ ID No:6 histone H1

histone H1 [Homo sapiens].
   ACCESSION CAA40409
   VERSION CAA40409.1 GI:296288
   DBSOURCE embl locus HSH11, accession X57130.1
   Eick,S., Nicolai,M., Mumberg,D. and Doenecke,D., Human H1 histones: conserved and varied sequence elements in two H1, subtype genes, Eur. J. Cell Biol. 49 (1), 110-115 (1989) PUBMED 2759094
Amino acid sequence including N-terminal methionine:

1 msetvppapa asaapekpla gkkakkpaka aaaskkkpag psvselivqa assskerggv
61 slaalkkala aagydveknn sriklgiksl vskgtlvqtk gtgasgsfkl nkkassvetk
121 pgaskvatkt katgaskklk katgaskksv ktpkkakkpa atrksskn pk kpktvkpkkv
181 akspakakav kpkaakarvt kpktakpkka apkkk

## Claims

1. A method of diagnosing a cancer, the method comprising the steps of:
(a) contacting a test tissue sample from an individual at risk of having a cancer or suspected of having a cancer or being evaluated for the possible presence of a cancer with an antibody that specifically binds to a modified histone protein; and
(b) determining the global H3K4 dimethylation and H3K18 acetylation histone modification pattern in the test tissue sample in comparison to a control tissue sample; thereby diagnosing said cancer by identification of an altered global histone modification pattern.

2. A method of providing a prognosis for a cancer, the method comprising the steps of:
(a) contacting a test tissue sample from an individual at risk for or known to have a cancer with an antibody that specifically binds to a modified histone protein; and
(b) determining the global H3K4 dimethylation and H3K18 acetylation histone modification pattern in the test tissue sample in comparison to a control tissue sample; thereby providing a prognosis for said cancer by identification of an altered global histone modification pattern.

3. A method of identifying a global histone modification pattern for the diagnosis or prognosis of cancer, said method comprising
(a) contacting a test tissue sample from an individual having the cancer with an antibody that specifically binds to a modified histone protein;
(b) contacting a control tissue sample from an individual not known to have the cancer with an antibody that specifically binds to the modified histone protein; and
(c) determining the global H3K4 dimethylation and H3K18 acetylation histone modification pattern of the modified histone protein in the test tissue sample in comparison to the control tissue sample; thereby identifying an altered global histone modification pattern useful in the diagnosis or prognosis of cancer.

4. A method of predicting the likelihood of a recurrence of cancer, said method comprising
(a) contacting a test tissue sample from the patient with an antibody that specifically binds to a modified histone protein; and
(b) determining the global H3K4 dimethylation and H3K18 acetylation histone modification pattern in the test tissue sample in comparison to a control tissue sample or nonmalignant tissue sample; thereby predicting the likelihood of a recurrence of cancer by identification of an altered global histone modification pattern.

5. The method of claims 1 to 4, wherein said tissue sample is a tumor biopsy sample.

6. The method of claims 1 to 4, wherein said cancer is prostate cancer.

7. The method of claims 1 to 4, wherein said cancer is bladder, kidney, ovarian, colon or breast cancer.

8. The method of claims 1 to 4, wherein said cancer is a metastatic cancer.

9. The method of claims 1 to 4, wherein said cancer is an adenocarcinoma.

10. The method of claims 1 to 4, wherein the cancer is a low grade or low stage cancer.

11. The method of claims 1 to 4, wherein said antibody is a monoclonal antibody.

12. A kit comprising at least two different antibodies which each selectively bind to a different histone modification which has a global histone modification pattern which is altered in cancer and instructions on how to relate the global histone modification to the diagnosis or prognosis of the cancer,
wherein the antibodies bind the H3 K4 dimethylation and H3K18 acetylation histon protein modifications.

## Patentansprüche

1. Ein Verfahren zur Krebsdiagnose, wobei das Verfahren Schritte umfasst, bei denen man:
(a) eine Test-Gewebeprobe von einem Individuum mit einem Risiko einen Krebs zu haben, oder von dem man annimmt, dass es einen Krebs hat, oder welches in Bezug auf die mögliche Anwesenheit von einem Krebs evaluiert wird, mit einem Antikörper in Kontakt bringt, der spezifisch an ein modifiziertes Histonprotein bindet; und
(b) die globalen Histon-Modifikationsmuster der H3K4 Dimethylierung und H3K18 Acetylierung in der Test-Gewebeprobe im Vergleich zu einer Kontroll-Gewebeprobe bestimmt; wodurch der Krebs durch Identifizierung eines veränderten globalen Histon-Modifikationsmusters diagnostiziert wird.

2. Ein Verfahren, eine Prognose bei einem Krebs zur Verfügung zu stellen, wobei das Verfahren die Schritte umfasst, bei denen man:
(a) eine Test-Gewebeprobe von einem Individuum mit einem Risiko für oder von dem man weiß, dass es einen Krebs hat, mit einem Antikörper in Kontakt bringt, der spezifisch an ein modifiziertes Histonprotein bindet; und
(b) die globalen Histon-Modifikationsmuster der H3K4 Dimethylierung und H3K18 Acetylierung in der Test-Gewebeprobe im Vergleich zu einer Kontroll-Gewebeprobe bestimmt; wodurch man eine Prognose für den Krebs durch Identifizierung eines veränderten globalen Histon-Modifikationsmusters zur Verfügung stellt.

3. Ein Verfahren zur Identifizierung eines globalen Histon-Modifikationsmusters zur Diagnose oder Prognose von Krebs, wobei das Verfahren Schritte umfasst, bei denen man:
(a) eine Test-Gewebeprobe von einem Individuum mit dem Krebs mit einem Antikörper in Kontakt bringt, welcher spezifisch an ein modifiziertes Histonprotein bindet;
(b) eine Kontroll-Gewebeprobe von einem Individuum, von dem man nicht weiß, ob es den Krebs hat, mit einem Antikörper in Kontakt bringt, der spezifisch an das modifizierte Histonprotein bindet; und
(c) die globalen Histon-Modifikationsmuster der H3K4 Dimethylierung und H3K18 Acetylierung in der Test-Gewebeprobe im Vergleich zu der Kontroll-Gewebeprobe bestimmt; wodurch man ein verändertes globales Histon-Modifikationsmuster nützlich zur Diagnose oder Prognose von Krebs identifiziert.

4. Ein Verfahren, um die Wahrscheinlichkeit eines Wiederauftretens von Krebs zu bestimmen, wobei das Verfahren Schritte umfasst, bei denen man
(a) eine Test-Gewebeprobe von dem Patienten mit einem Antikörper in Kontakt bringt, der spezifisch an ein modifiziertes Histonprotein bindet; und
(b) die globalen Histon-Modifikationsmuster der H3K4 Dimethylierung und H3K18 Acetylierung in der Test-Gewebeprobe im Vergleich zu einer Kontroll-Gewebeprobe oder einer nichtmalignen Gewebeprobe bestimmt; wodurch man die Wahrscheinlichkeit eines Wiederauftretens von Krebs durch Identifizierung eines veränderten globalen Histon-Modifikationsmusters vorhersagt.

5. Das Verfahren nach Ansprüchen 1 bis 4, **dadurch gekennzeichnet, dass** die Gewebeprobe eine Tumorbiopsieprobe ist.

6. Das Verfahren nach Ansprüchen 1 bis 4, **dadurch gekennzeichnet, dass** der Krebs Prostatakrebs ist.

7. Das Verfahren nach Ansprüchen 1 bis 4, **dadurch gekennzeichnet, dass** der Krebs Blasen-, Nieren-, Ovar-, Kolon- oder Brustkrebs ist.

8. Das Verfahren nach Ansprüchen 1 bis 4, **dadurch gekennzeichnet, dass** der Krebs ein metastatischer Krebs ist.

9. Das Verfahren nach Ansprüchen 1 bis 4, **dadurch gekennzeichnet, dass** der Krebs ein Adenocarcinom ist.

10. Das Verfahren nach Ansprüchen 1 bis 4, **dadurch gekennzeichnet, dass** der Krebs ein Krebs unteren Grades oder unteren Stadiums ist.

11. Das Verfahren nach Ansprüchen 1 bis 4, **dadurch gekennzeichnet, dass** der Antikörper ein monoklonaler Antikörper ist.

12. Ein Kit, umfassend mindestens zwei verschiedene Antikörper, welche jeweils selektiv an eine unterschiedliche Histon-Modifikation binden, welche ein globales Histon-Modifikationsmuster aufweist, welches bei Krebs geändert ist, und Instruktionen dazu, wie man die globale Histon-Modifikation mit der Diagnose oder Prognose des Krebses in Bezug setzt, **dadurch gekennzeichnet, dass** die Antikörper die Histonprotein-Modifikationen H3K4 Dimethylierung und H3K18 Acetylierung binden.

## Revendications

1. Procédé permettant de diagnostiquer un cancer, lequel procédé comporte les étapes suivantes :
a) mettre un échantillon de tissu à tester, provenant d'un individu risquant d'être atteint d'un cancer, suspecté d'être atteint d'un cancer ou examiné en vue de détecter la présence éventuelle d'un cancer, en contact avec un anticorps qui se lie spécifiquement à une protéine histone modifiée ;
b) et déterminer le schéma global de modification d'histone par diméthylation H3K4 et acétylation H3K18 dans l'échantillon de tissu testé, en comparaison d'un échantillon de tissu témoin ;
ce qui permet de diagnostiquer un cancer par identification d'une altération du schéma global de modification d'histone.

2. Procédé permettant de faire le pronostic d'un cancer, lequel procédé comporte les étapes suivantes :
a) mettre un échantillon de tissu à tester, provenant d'un individu risquant d'être atteint d'un cancer ou dont on sait qu'il est atteint d'un cancer, en contact avec un anticorps qui se lie spécifiquement à une protéine histone modifiée ;
b) et déterminer le schéma global de modification d'histone par diméthylation H3K4 et acétylation H3K18 dans l'échantillon de tissu testé, en comparaison d'un échantillon de tissu témoin ;
ce qui permet de faire le pronostic dudit cancer par identification d'une altération du schéma global de modification d'histone.

3. Procédé permettant d'identifier un schéma global de modification d'histone en vue de faire le diagnostic ou le pronostic d'un cancer, lequel procédé comporte les étapes suivantes :
a) mettre un échantillon de tissu à tester, provenant d'un individu atteint d'un cancer, en contact avec un anticorps qui se lie spécifiquement à une protéine histone modifiée ;
b) mettre un échantillon de tissu témoin, provenant d'un individu qu'on sait ne pas être atteint de ce cancer, en contact avec un anticorps qui se lie spécifiquement à la protéine histone modifiée ;
c) et déterminer le schéma global de modification d'histone par diméthylation H3K4 et acétylation H3K18 dans l'échantillon de tissu testé, en comparaison de l'échantillon de tissu témoin ;
ce qui permet d'identifier une altération du schéma global de modification d'histone, utile pour faire le diagnostic ou le pronostic de ce cancer.

4. Procédé permettant de prédire la probabilité de rechute d'un cancer, lequel procédé comporte les étapes suivantes :
a) mettre un échantillon de tissu à tester, provenant du patient, en contact avec un anticorps qui se lie spécifiquement à une protéine histone modifiée ;
b) et déterminer le schéma global de modification d'histone par diméthylation H3K4 et acétylation H3K18 dans l'échantillon de tissu testé, en comparaison d'un échantillon de tissu témoin ou d'un échantillon de tissu non malin ;
ce qui permet de prédire la probabilité de rechute du cancer par identification d'une altération du schéma global de modification d'histone.

5. Procédé conforme à l'une des revendications 1 à 4, dans lequel ledit échantillon de tissu est un échantillon issu d'une biopsie de tumeur.

6. Procédé conforme à l'une des revendications 1 à 4, dans lequel ledit cancer est un cancer de la prostate.

7. Procédé conforme à l'une des revendications 1 à 4, dans lequel ledit cancer est un cancer de la vessie, du rein, des ovaires, du côlon ou du sein.

8. Procédé conforme à l'une des revendications 1 à 4, dans lequel ledit cancer est un cancer métastatique.

9. Procédé conforme à l'une des revendications 1 à 4, dans lequel ledit cancer est un adénocarcinome.

10. Procédé conforme à l'une des revendications 1 à 4, dans lequel le cancer est un cancer de bas grade ou un cancer de bas stade.

11. Procédé conforme à l'une des revendications 1 à 4, dans lequel ledit anticorps est un anticorps monoclonal.

12. Trousse comprenant au moins deux anticorps différents qui se lient sélectivement chacun à une histone modifiée différente qui présente un schéma global de modification d'histone qui se trouve altéré en cas de cancer, ainsi que des instructions indiquant comment relier le schéma global de modification d'histone à l'établissement du diagnostic ou du pronostic d'un cancer, lesquels anticorps se lient aux protéines histones modifiées par diméthylation H3K4 et par acétylation H3K18.
